# EUROPÄISCHE PATENTSCHRIFT

(11) **EP 2 934 466 B1**
(45) Veröffentlichungstag und Bekanntmachung des Hinweises auf die Patenterteilung: **06.02.2019**
(21) Anmeldenummer: 13811168.7
(22) Anmeldetag: 16.12.2013
(51) Int. Cl.: A61K 8/46, A61K 8/26, A61Q 15/00

(54) **TEXTIL SCHONENDE NONAEROSOL-ANTITRANSPIRANTIEN MIT METHANSULFONSÄURE**
TEXTILE-SPARING, NON-AEROSOL ANTIPERSPIRANTS WITH METHANESULPHONIC ACID
ANTITRANSPIRANTS NON AÉROSOLS MÉNAGEANT LES TEXTILES, CONTENANT DE L'ACIDE MÉTHANE-SULFONIQUE

(30) Priorität: 21.12.2012 DE 102012224133
(43) Veröffentlichungstag der Anmeldung: 28.10.2015
(73) Patentinhaber: Henkel AG & Co. KGaA, 40589 Düsseldorf (DE)
(72) Erfinder: BANOWSKI, Bernhard, 40597 Düsseldorf (DE); CLAAS, Marcus, 40723 Hilden (DE)
(86) Internationale Anmeldenummer: PCT/EP2013/076656
(87) Internationale Veröffentlichungsnummer: WO 2014/095688

(56) Entgegenhaltungen:
- WO-A1-2011/050044
- WO-A2-2010/097205
- DE-A1- 2 249 580
- US-A- 3 953 450
- US-A- 4 110 428
- Paul S. Tully: "Sulfonic Acids", Kirk-Othmer Encyclopedia of Chemical Technology, 4. Dezember 2000 (2000-12-04), XP055134265, DOI: 10.1002/0471238961.1921120620211212.a01 Gefunden im Internet: URL:http://onlinelibrary.wiley.com/store/1 0.1002/0471238961.1921120620211212.a01/ass et/sulftull.a01.pdf?v=1&t=hyqu3ve2&s=c218f da68a537c12c64744e2e3edb4fdaa473006 [gefunden am 2014-08-12]

## Beschreibung

Die vorliegende Anmeldung betrifft kosmetische Antitranspirantien, die eine geringere Textilanschmutzung aufweisen als bekannte Antitranspirantien.

Das Waschen, Reinigen und Pflegen des eigenen Körpers stellt ein menschliches Grundbedürfnis dar, und die moderne Industrie versucht fortlaufend, diesen Bedürfnissen des Menschen in vielfältiger Weise gerecht zu werden. Besonders wichtig für die tägliche Hygiene ist die anhaltende Beseitigung oder zumindest Reduzierung des Körpergeruchs. Im Stand der Technik sind zahlreiche spezielle deodorierende oder schweißhemmende Körperpflegemittel bekannt, die für die Anwendung in Körperregionen mit einer hohen Dichte von Schweißdrüsen, insbesondere im Achselbereich, entwickelt wurden. Diese sind in den unterschiedlichsten Darreichungsformen konfektioniert, beispielsweise als Puder, in Stiftform, als Aerosolspray, Pumpspray, flüssige und gelförmige Roll on-Applikation, Creme, Gel und als getränktes flexibles Substrat (Deotücher).

Kosmetische Antitranspirantien enthalten auf jeden Fall mindestens ein schweißhemmendes Salz. Meistens sind noch mindestens ein Öl oder eine Fettsubstanz und auf jeden Fall immer eine Riechstoffkomponente bzw. ein Parfüm enthalten.

Bei regelmäßiger Anwendung können Antitranspirantien zu deutlich sichtbaren farbigen Textilanschmutzungen führen. Oft handelt es sich hier um gelbe Flecken, die auch durch intensives Waschen nicht entfernt werden können. Die Fleckenbildung basiert auf einer komplexen Interaktion von Rezepturbestandteilen, Schweiß und dem verwendeten Waschmittel. Wahrscheinlich bilden sich zunächst unlösliche Aluminiumverbindungen auf und innerhalb der Faser. Die Gelbfärbung tritt in der Regel mit einer Zeitverzögerung ein und wird zumindest teilweise durch Oxidation ungesättigter Fettsäuren hervorgerufen, die als unlösliche Aluminiumsalze vorliegen. Leider können hier unterschiedliche Faktoren in unerwarteter Weise zusammenwirken und je nach Wahl des Parfumöls, des Waschmittels und je nach individueller Schweißmenge und -zusammensetzung können sich ausgeprägte gelbe Flecken auf Textilien bilden.

Durch die Interaktion von Waschmitteln und Antitranspirantwirkstoffen entstehen unlösliche Verbindungen, die auf ein Textil aufziehen können. Diese unlöslichen Verbindungen bilden weiße, harte Rückstände, die sich meist erst nach mehreren Anschmutz und Waschzyklen auf dem Textil zeigen. Diese weißen Rückstände sind in Wasser nicht löslich und sind auch durch ein Standard-Waschverfahren nicht zu entfernen. Sie sind besonders gut auf leicht oder dunkel gefärbten Textilien erkennbar. Die geschickte Wahl von Additiven führt zu einer deutlich geringeren bzw. Verzögerung der Bildung dieser unlöslichen Ablagerungen.

Zur Maskierung von weißen Rückständen auf dunklen Textilien, z. B. durch Transfer der Produkte von der Haut auf ein Textil beim Anziehen, werden kosmetische Öle oder Polyole eingesetzt. Diese Maskierungsmittel können ebenfalls auf ein Textil aufziehen. Je nach chemischer Zusammensetzung lassen sich diese Maskierungsmittel nur teilweise oder gar nicht durch einen Standardwaschprozess entfernen. Das hydrophobe Maskierungsmittel reichert sich auf dem Textil an und führt zu einem dunklen, fettigen/öligen Fleck, der u. a. auch die Haptik der Textilien im angeschmutzen Bereich verändern kann. Die geschickte Wahl von Additiven führt zu einer deutlich geringeren bzw. Verzögerung der Bildung dieser öliger/fettigen, dunklen Anschmutzungen.

Es besteht daher ein Bedarf an Antitranspirant-Rezepturen, die zuverlässig die Bildung gelber, weißer und/oder fettiger Flecken verhindern können.

Um Textilien gegenüber derartigen dauerhaften Verschmutzungen zu schützen, werden im Stand der Technik diverse Inhaltsstoffe zugesetzt. Ein häufig verwendeter Zusatz sind Tenside, wie z. B. offenbart in WO 2010/097205 A2. Auch die Wahl der Ölkomponenten kann die Textilanschmutzung verringern oder eben erhöhen, siehe zum Beispiel US 5925338, US 4511554 oder US 3974270.

US 3953450 A offenbart Antitranspirantzusammensetzungen, die als schweißhemmenden Wirkstoff Bis-(2-N-Oxopyridyl-2-thio)aluminiumverbindungen, darunter Bis-(2-N-Oxopyridyl-2-thio)aluminiummethansulfonat, enthalten. DE 2249580 A offenbart Antitranspirantzusammensetzungen, die als schweißhemmenden Wirkstoff ausgewählte heterozyklische Aluminiumverbindungen, darunter die Verbindung 2-Methansulfonyloxy-5,5-bis(ethoxycarbonyl)-1,3,2-dioxalumanne, enthalten.

Die Aufgabe der vorliegenden Erfindung bestand darin, kosmetische Antitranspirantien bereitzustellen, die ein schweißhemmendes Aluminiumsalz enthalten und nicht oder nur in stark reduziertem Umfang zu bleibenden Textilverfärbungen führen.

Überraschend wurde nun gefunden, dass ein Zusatz von Methansulfonsäure die gestellten Aufgaben sehr gut löst und sich hervorragend als Verfärbungsinhibitor für den Einsatz in kosmetischen Antitranspirantien eignet.

Ein erster Gegenstand der vorliegenden Anmeldung ist daher ein schweißhemmendes kosmetisches Mittel zur Nonaerosol-Anwendung, enthaltend in einem kosmetisch verträglichen Träger
a) mindestens ein schweißhemmendes Aluminiumsalz in einer Gesamtmenge von 2 - 40 Gew.-%, bevorzugt 8 - 35 Gew.-%, besonders bevorzugt 10 - 28 Gew.-% und außerordentlich bevorzugt 12 - 20 Gew.-%, wobei sich die Gew.-%-Angaben auf das Gesamtgewicht der kristallwasserfreien und ligandenfreien Aktivsubstanz (USP) in dem Mittel beziehen, und zusätzlich dazu
b) Methansulfonsäure der folgenden Formel (MS-1) 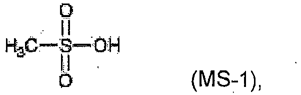 wobei das schweißhemmende Aluminiumsalz ausgewählt ist aus den wasserlöslichen adstringierenden anorganischen Salzen von Aluminium und Aluminium-Zirconium-Mischungen.

Ein weiterer Gegenstand der vorliegenden Anmeldung ist die Verwendung von Methansulfonsäure der folgenden Formel (MS-1), 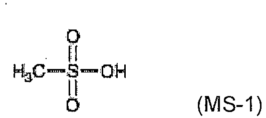 und/oder mindestens einem physiologisch verträglichen Salz der Methansulfonsäure in einem schweißhemmenden kosmetischen Mittel, enthaltend in einem kosmetisch verträglichen Träger mindestens ein schweißhemmendes Aluminiumsalz, das bevorzugt Zirconium-frei ist, in einer Gesamtmenge von 2 - 40 Gew.-%, bevorzugt 8 - 35 Gew.-%, besonders bevorzugt 10 - 28 Gew.-% und außerordentlich bevorzugt 12 - 20 Gew.-%, wobei sich die Gew.-%-Angaben auf das Gesamtgewicht der kristallwasserfreien und ligandenfreien Aktivsubstanz (USP) in der Zusammensetzung beziehen, zur Reduzierung oder Verhinderung von durch . Antitranspirantien und Antitranspirantwirkstoffe verursachten Textilverfärbungen und/ oder Textilflecken, wobei sich die Gew.-%-Angaben jeweils auf das Gesamtgewicht des Mittels beziehen.

Bezüglich bevorzugter Ausführungsformen der erfindungsgemäßen Verwendung gilt mutatis mutandis das im Folgenden zu den erfindungsgemäßen Mitteln Gesagte.

Weitere bevorzugte erfindungsgemäße Mittel sind dadurch gekennzeichnet, dass Methansulfonsäure in einer Gesamtmenge von 0,05 - 5 Gew.-%, bevorzugt 0,1 - 3 Gew.-%, besonders bevorzugt 0,5 - 2,5 Gew.-%, außerordentlich bevorzugt 1 - 2 Gew.-%, enthalten ist, jeweils bezogen auf das Gesamtgewicht des Mittels, wobei die Gesamtmenge an Methansulfonsäure und/oder mindestens einem physiologisch verträglichen Salz der Methansulfonsäure 0,05 - 5 Gew.-%, bevorzugt 0,1 - 3 Gew.-%, besonders bevorzugt 0,5 - 2,5 Gew.-%, außerordentlich bevorzugt 1 - 2 Gew.-%, jeweils bezogen auf das Gesamtgewicht des Mittels, beträgt.

Es sei an dieser Stelle darauf hingewiesen, dass sich die Angabe Gew.-%, sofern es nicht anders angegeben ist, jeweils auf das Gesamtgewicht des erfindungsgemäßen Mittels bezieht.

Die Begriffe "erfindungsgemäßes Mittel" und "erfindungsgemäße Zusammensetzung" werden in der vorliegenden Anmeldung synonym gebraucht.

"Normalbedingungen" sind im Sinne der vorliegenden. Anmeldung eine Temperatur von 20 °C und ein Druck von 1013 mbar. Schmelzpunktangaben beziehen sich ebenfalls auf einen Druck von 1013 mbar.

Die erfindungsgemäßen Mittel enthalten einen kosmetisch verträglichen Träger. Erfindungsgemäß bevorzugt ist der kosmetisch verträgliche Träger unter Normalbedingungen (20°C, 1013 mbar) flüssig oder fest. Weitere erfindungsgemäß bevorzugte kosmetisch verträgliche Träger umfassen mindestens ein kosmetisches Öl, das kein Riechstoff und kein ätherisches Öl ist.

Die unter Normalbedingungen flüssigen kosmetischen Öle sind mit Wasser nicht mischbar.

Sofern in der vorliegenden Anmeldung von einem kosmetischen Öl die Rede ist, handelt es sich hierbei immer um ein kosmetisches Öl, das kein Riechstoff und kein ätherisches Öl ist, unter Normalbedingungen flüssig und mit Wasser nicht mischbar ist.

In einer ersten bevorzugten Ausführungsform der Erfindung enthält das erfindungsgemäße Mittel null bis maximal 10 Gew.-% freies Wasser, bevorzugt null bis maximal 5 Gew.-% freies Wasser. Der Gehalt an Kristallwasser, Hydratationswasser oder ähnlich molekular gebundenem Wasser, der in den eingesetzten Bestandteilen, insbesondere in den schweißhemmenden Wirkstoffen, enthalten ist, stellt im Sinne der vorliegenden Anmeldung kein freies Wasser dar und wird daher bei der Berechnung der Wassermenge nicht berücksichtigt.

Überraschend wurde festgestellt, dass der Textilverfärbungs-reduzierende oder -verhindernde Effekt der Methansulfonsäure besonders gut hervortritt in Mitteln, die 15 - 96 Gew.-% freies Wasser enthalten.

In einer besonders bevorzugten Ausführungsform der Erfindung enthält das erfindungsgemäße Mittel daher freies Wasser in einer Gesamtmenge von 15 - 96 Gew.-%, bevorzugt 25 - 80 Gew.-%, besonders bevorzugt 30 - 70 Gew.-%, außerordentlich bevorzugt 40 - 60 Gew.-%, jeweils bezogen auf das Gesamtgewicht des erfindungsgemäßen Mittels.

### Antitranspirant-Wirkstoffe

Die erfindungsgemäßen Zusammensetzungen enthalten als Antitranspirant-Wirkstoff mindestens ein schweißhemmendes Aluminiumsalz in einer Gesamtmenge von 2 - 40 Gew.-%, bevorzugt 8 - 35 Gew.-%, besonders bevorzugt 10 - 28 Gew.-% und außerordentlich bevorzugt 12 - 20 Gew.-%, wobei sich die Gew.-%-Angaben auf das Gesamtgewicht der kristallwasserfreien und ligandenfreien Aktivsubstanz (USP) in der Zusammensetzung beziehen.

Es kann erfindungsgemäß bevorzugt sein, dass das kosmetische Mittel frei ist von Zirconium-Verbindungen.

Die schweißhemmenden Aluminiumsalze sind ausgewählt aus den wasserlöslichen adstringierenden anorganischen Salzen von Aluminium und Aluminium-Zirconium-Mischungen. Alumosilicate und Zeolithe zählen erfindungsgemäß nicht zu den Antitranspirant-Wirkstoffen. Erfindungsgemäß wird unter Wasserlöslichkeit eine Löslichkeit von wenigstens 3 Gew.-% bei 20 °C verstanden, das heißt, dass Mengen von wenigstens 3 g des Antitranspirant-Wirkstoffs in 97 g Wasser bei 20 °C löslich sind.

Besonders bevorzugte Antitranspirant-Wirkstoffe sind ausgewählt aus Aluminiumchlorhydrat, insbesondere Aluminiumchlorhydrat mit der allgemeinen Formel [Al₂(OH)₅Cl · 1-6 H₂O]ₙ, bevorzugt [Al₂(OH)₅Cl · 2-3 H₂O]ₙ, das in nicht-aktivierter oder in aktivierter (depolymerisierter) Form vorliegen kann, sowie Aluminiumchlorhydrat mit der allgemeinen Formel [Al₂(OH)₄Cl₂ · 1-6 H₂O]ₙ, bevorzugt [Al₂(OH)₄Cl₂ · 2-3 H₂O]ₙ, das in nicht-aktivierter oder in aktivierter (depolymerisierter) Form vorliegen kann.

Die Herstellung bevorzugter Antitranspirant-Wirkstoffe ist beispielsweise in US 3887692, US 3904741, US 4359456, GB 2048229 und GB 1347950 offenbart.

Weiterhin bevorzugt sind Aluminiumsesquichlorhydrat, Aluminiumdichlorhydrat, Aluminiumchlorhydrex-Propylenglykol (PG) oder Aluminiumchlorhydrex-Polyethylenglykol (PEG), Aluminium- oder Aluminiumzirkonium-Glycol-Komplexe, z. B. Aluminium- oder Aluminiumzirkonium-Propylenglycol-Komplexe, Aluminiumsesquichlorhydrex-PG oder Aluminiumsesquichlorhydrex-PEG, Aluminium-PG-dichlorhydrex oder Aluminium-PEG-dichlorhydrex, Aluminiumhydroxid, weiterhin ausgewählt aus den Aluminiumzirconiumchlorhydraten, wie Aluminiumzirconiumtrichlorhydrat, Aluminiumzirconiumtetrachlorhydrat, Aluminiumzirconiumpehtachlorhydrat, Aluminiumzirconiumoctachlorhydrat, den Aluminium-Zirconium-Chlorohydrat-Glycin-Komplexen wie Aluminiumzirconiumtrichlorhydrexglycin, Aluminiumzirconiumtetrachlorhydrexglycin, Aluminiumzirconiumpentachlorhydrexglycin, Aluminiumzirconiumoctachlorhydrexglycin, Kaliumaluminiumsulfat (KAl(SO₄)₂ · 12 H₂O, Alaun), Aluminiumundecylenoylcollagenaminosäure, Natriumaluminiumlactat + Aluminiumsulfat, Natriumaluminiumchlorhydroxylactat, Aluminiumbromhydrat, Aluminiumchlorid, den Aluminiumsalzen von Lipoaminosäuren, Aluminiumsulfat, Aluminiumlactat, Aluminiumchlorhydroxyallantoinat und Natrium-Aluminium-Chlorhydroxylactat.

Erfindungsgemäß besonders bevorzugte Antitranspirant-Wirkstoffe sind ausgewählt aus so genannten "aktivierten" Aluminium- und Aluminium-Zirconiumsalzen, die auch als Antitranspirant-Wirkstoffe "mit erhöhter Wirksamkeit (englisch: enhanced activity)" bezeichnet werden. Derartige Wirkstoffe sind im Stand der Technik bekannt und auch kommerziell erhältlich. Ihre Herstellung ist beispielsweise in GB 2048229, US 4775528 und US 6010688 offenbart. Aktivierte Aluminium- und Aluminium-Zirconiumsalze werden in der Regel durch Wärmebehandlung einer relativ verdünnten Lösung des Salzes erzeugt (z.B. etwa 10 Gew.-% Salz), um dessen HPLC-Peak 4-zu-Peak 3-Flächenverhältnis zu vergrößern. Das aktivierte Salz kann anschließend zu einem Pulver getrocknet, insbesondere sprühgetrocknet werden. Neben der Sprühtrocknung ist z. B. auch die Walzentrocknung geeignet.

Aktivierte Aluminium- und Aluminium-Zirconiumsalze haben typischerweise ein HPLC-Peak 4-zu-Peak 3-Flächenverhältnis von mindestens 0,4, bevorzugt mindestens 0,7, besonders bevorzugt mindestens 0,9, wobei mindestens 70% des Aluminiums diesen Peaks zuzuordnen sind.

Aktivierte Aluminium- und Aluminium-Zirconiumsalze müssen nicht notwendigerweise als sprühgetrocknetes Pulver eingesetzt werden. Erfindungsgemäß ebenfalls bevorzugte schweißhemmende Wirkstoffe sind nicht-wässrige Lösungen oder Solubilisate eines aktivierten schweißhemmenden Aluminium- oder Aluminium-Zirconiumsalzes, beispielsweise gemäß US 6010688, die durch den Zusatz einer wirksamen Menge eines mehrwertigen Alkohols, der 3 bis 6 Kohlenstoffatome und 3 bis 6 Hydroxyl-Gruppen, bevorzugt Propylenglycol, Sorbit und Pentaerythrit, aufweist, gegen den Verlust der Aktivierung gegen den raschen Abbau des HPLC-Peak 4:Peak 3-Flächenverhältnisses des Salzes stabilisiert sind. Beispielsweise bevorzugt sind Zusammensetzungen, die in Gewichtsprozent (USP) enthalten: 18 - 45 Gew.-% eines aktivierten Aluminium- oder Aluminium-Zirconiumsalzes, 55 - 82 Gew.-% mindestens eines wasserfreien mehrwertigen Alkohols mit 3 bis 6 Kohlenstoffatomen und 3 bis 6 Hydroxyl-Gruppen, bevorzugt 1,2-Propylenglycol, 1,3-Butylenglycol, Diethylenglycol, Dipropylenglycol, Glycerin, Sorbit und Pentaerythrit, besonders bevorzugt 1,2-Propylenglycol.

Besonders bevorzugt sind auch Komplexe aktivierter schweißhemmender Aluminium- oder Aluminium-Zirconiumsalze mit einem mehrwertigen Alkohol, die 20 - 50 Gew.-%, besonders bevorzugt 20 - 42 Gew.-%, aktiviertes schweißhemmendes Aluminium- oder Aluminium-Zirconiumsalz und 2 - 16 Gew.-% molekular gebundenes Wasser enthalten, wobei der Rest zu 100 Gew.-% mindestens ein mehrwertiger Alkohol mit 3 bis 6 Kohlenstoffatome und 3 bis 6 Hydroxyl-Gruppen ist. Propylenglycol, Propylenglycol/Sorbit-Mischungen und Propylenglycol/Pentaerythrit-Mischungen sind bevorzugte derartige Alkohole. Derartige erfindungsgemäß bevorzugte Komplexe eines aktivierten schweißhemmenden Aluminium- oder Aluminium-Zirconiumsalzes mit einem mehrwertigen Alkohol sind z. B. offenbart in US 5643558 und US 6245325.

Weitere bevorzugte schweißhemmende Wirkstoffe sind basische Calcium-Aluminiumsalze, wie sie z. B. in US 2571030 offenbart sind. Diese Salze werden durch Umsetzen von Calciumcarbonat mit Aluminiumchlorhydroxid oder Aluminiumchlorid und Aluminiumpulver oder durch Zusetzen von Calciumchlorid-Dihydrat zu Aluminiumchlorhydroxid hergestellt.

Weitere bevorzugte schweißhemmende Wirkstoffe sind Aluminium-Zirconium-Komplexe, wie sie z. B. in US 4017599 offenbart sind, die mit Salzen von Aminosäuren, insbesondere mit Alkali- und Erdalkaliglycinaten, gepuffert sind.

Weitere bevorzugte schweißhemmende Wirkstoffe sind aktivierte Aluminium- oder Aluminium-Zirconiumsalze, wie sie beispielsweise in US 6245325 oder US 6042816 offenbart sind, enthaltend 5 - 78 Gew.-% (USP) eines aktivierten schweißhemmenden Aluminium- oder Aluminium-Zirconiumsalzes, eine Aminosäure oder Hydroxyalkansäure in einer solchen Menge, um ein (Aminosäure oder Hydroxyalkansäure) zu (Al+Zr) - Gewichtsverhältnis von 2:1 - 1:20 und bevorzugt 1:1 bis 1:10 bereitzustellen, sowie ein wasserlösliches Calciumsalz in einer solchen Menge, um ein Ca:(Al+Zr)-Gewichtsverhältnis von 1:1 - 1:28 und bevorzugt 1:2 - 1:25 bereitzustellen. Besonders bevorzugte feste aktivierte schweißhemmende Salzzusammensetzungen, beispielsweise gemäß US 6245325 oder US 6042816, enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser (Hydratationswasser), weiterhin soviel wasserlösliches Calciumsalz, dass das Ca:(Al+Zr)-Gewichtsverhältnis 1:1 - 1:28, bevorzugt 1:2 - 1:25, beträgt und soviel Aminosäure, dass das Aminosäure zu (Al+Zr) - Gewichtsverhältnis 2:1 - 1:20, bevorzugt 1:1 - 1:10, beträgt.

Weitere besonders bevorzugte feste schweißhemmende aktivierte Salzzusammensetzungen, z. B. gemäß US 6245325 oder US 6042816, enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser (Hydratationswasser), weiterhin soviel wasserlösliches Calciumsalz, dass das Ca:(Al+Zr)-Gewichtsverhältnis 1:1 - 1:28, bevorzugt 1:2 - 1:25, beträgt und soviel Glycin, dass das Glycin zu (Al+Zr) - Gewichtsverhältnis 2:1 - 1:20, bevorzugt 1:1 - 1:10, beträgt.

Weitere besonders bevorzugte feste schweißhemmende aktivierte Salzzusammensetzungen, z. B. gemäß US 6245325 oder US 6042816, enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser, weiterhin soviel wasserlösliches Calciumsalz, dass das Ca:(Al+Zr)-Gewichtsverhältnis 1:1 - 1:28, bevorzugt 1:2 - 1:25, beträgt und soviel Hydroxyalkansäure, dass das Hydroxyalkansäure zu (Al+Zr) - Gewichtsverhältnis 2:1 - 1:20, bevorzugt 1:1 - 1:10, beträgt.

Für die Stabilisierung der schweißhemmenden Salze bevorzugte Aminosäuren sind ausgewählt aus Glycin, Alanin, Leucin, Isoleucin, β-Alanin, Valin, Cystein, Serin, Tryptophan, Phenylalanin, Methionin, β-Amino-n-butansäure und γ-Amino-n-butansäure und den Salzen davon, jeweils in der d-Form, der I-Form und der dl-Form; Glycin ist besonders bevorzugt.

Für die Stabilisierung der schweißhemmenden Salze bevorzugte Hydroxyalkansäuren sind ausgewählt aus Glycolsäure und Milchsäure.

Weitere bevorzugte aktivierte Aluminiumsalze sind solche der allgemeinen Formel Al₂(OH)₆₋ₐXa, worin X Cl, Br, I oder NO₃ ist und "a" ein Wert von 0,3 bis 5, bevorzugt von 0,8 bis 2,5 und besonders bevorzugt 1 bis 2 ist, so dass das Molverhältnis von AI:X 0,9:1 bis 2,1:1 beträgt, wie sie beispielsweise in US 6074632 offenbart sind. Bei diesen Salzen ist im Allgemeinen etwas Hydratationswasser assoziativ gebunden, typischerweise 1 bis 6 Mol Wasser pro Mol Salz. Besonders bevorzugt ist Aluminiumchlorhydrat (d.h. X ist Cl in der vorgenannten Formel) und speziell 5/6-basisches Aluminiumchlorhydrat, worin "a" 1 beträgt, so dass das Molverhältnis von Aluminium zu Chlor, 1,9:1 bis 2,1:1 beträgt.

Bevorzugte aktivierte Aluminium-Zirconiumsalze sind solche, die Mischungen oder Komplexe der vorstehend beschriebenen Aluminiumsalze mit Zirconiumsalzen der Formel ZrO(OH)_{2-pb}Y_{b} darstellen, worin Y Cl, Br, I, NO₃ oder SO₄ ist, b eine rationale Zahl von 0,8 bis 2 und p die Wertigkeit von Y ist, wie sie beispielsweise in US 6074632 offenbart sind. Die Zirconiumsalze haben in der Regel ebenfalls etwas Hydratationswasser assoziativ gebunden, typischerweise 1 bis 7 Mol Wasser pro Mol Salz. Vorzugsweise ist das Zirconiumsalz Zirconylhydroxychlorid mit der Formel ZrO(OH)_{2-b}Cl_{b}, worin b eine rationale Zahl von 0,8 bis 2, bevorzugt 1,0 bis 1,9 ist. Bevorzugte Aluminium-Zirconiumsalze haben ein Al:Zr-Molverhältnis von 2 bis 10 und ein Metall:(X+Y)-Verhältnis von 0,73 bis 2,1, bevorzugt 0,9 bis 1,5. Ein besonders bevorzugtes Salz ist Aluminium-Zirconiumchlorhydrat (d.h., X und Y sind Cl), das ein Al:Zr-Verhältnis von 2 bis 10 und ein molares Metall:Cl-Verhältnis von 0,9 bis 2,1 hat. Der Begriff Aluminium-Zirconiumchlorhydrat umfasst die Tri-, Tetra-, Penta- und Octachlorhydratformen. Weitere bevorzugte schweißhemmende Wirkstoffe sind in US 6663854 und US 20040009133 offenbart.

Bevorzugte schweißhemmende Aluminium-Zirconium-Salze weisen ein molares Metall-zu-Chlorid-Verhältnis von 0,9 - 1,5, bevorzugt 0,9 - 1,3, besonders bevorzugt 0,9 - 1,1, auf.

Erfindungsgemäß besonders bevorzugte Zirconium-freie Aluminiumsalze weisen ein molares Metall-zu-Chlorid-Verhältnis von 1,9 - 2,1 auf. Erfindungsgemäß besonders bevorzugte Zirconium-freie Aluminiumsesquichlorohydrate weisen ein molares Metall-zu-Chlorid-Verhältnis von 1,5:1-1,8:1 auf. Bevorzugte Aluminiumzirconiumchlorohydrate haben im allgemeinen die empirische Formel AlₙZr(OH)_{[3n+4-m(n+1)]}(Cl)_{[m(n+1)]} mit n = 2,0 - 10,0, bevorzugt 3,0 - 8,0, m = 0,77 - 1,11 (entsprechend einem molaren Metall (Al+Zr)-zu-Chlorid-Verhältnis von 1,3 - 0,9), bevorzugt m = 0,91 - 1,11 (entsprechend M:CI = 1,1 - 0,9), und besonders bevorzugt m = 1,00 - 1,11 (entsprechend M:Cl = 1,0 - 0,9), weiterhin sehr bevorzugt m = 1,02 - 1,11 (entsprechend M:Cl = 0,98 - 0,9) sowie sehr bevorzugt m = 1,04 - 1,11 (entsprechend M:Cl = 0,96 - 0,9). Bei diesen Salzen ist im Allgemeinen etwas Hydratationswasser assoziativ gebunden, typischerweise 1 - 6 Mol Wasser pro Mol Salz, entsprechend 1 - 16 Gew.-%, bevorzugt 4 - 13 Gew.-% Hydratationswasser.

Üblicherweise sind die bevorzugten Aluminiumzirconiumchlorohydrate mit einer Aminosäure assoziiert, um die Polymerisation der Zirconiumspecies während der Herstellung zu verhindern. Bevorzugte stabilisierende Aminosäuren sind ausgewählt aus Glycin, Alanin, Leucin, Isoleucin, β-Alanin, Cystein, Valin, Serin, Tryptophan, Phenylalanin, Methionin, β-Amino-n-butansäure und γ-Amino-n-butansäure und den Salzen davon, jeweils in der d-Form, der I-Form und der dl-Form; Glycin ist besonders bevorzugt. Die Aminosäure ist in einer Menge von 1 - 3 Mol, bevorzugt 1,3 - 1,8 Mol, jeweils pro Mol Zirconium in dem Salz enthalten.

Bevorzugte schweißhemmende Salze sind Aluminium-Zirconiumtetrachlorohydrate (Al:Zr = 2-6; M:Cl = 0.9-1.3), insbesondere Salze mit einem molaren Metall-zu-Chlorid-Verhältnis von 0,9 - 1,1, bevorzugt 0,9 - 1,0.

In einer besonders bevorzugten erfindungsgemäßen Ausführungsform ist als besonders wirksames Antitranspirant-Salz ein so genanntes "aktiviertes" Salz enthalten, insbesondere eines mit einem hohen HPLC-Peak 5-Aluminium-Gehalt, insbesondere mit einer Peak 5-Fläche von mindestens 33 %, besonders bevorzugt mindestens 45 %, bezogen auf die gesamte Fläche unter den Peaks 2 - 5, gemessen mit HPLC einer 10 Gew.-%igen wässrigen Lösung des Wirkstoffs unter Bedingungen, bei denen die Aluminiumspecies in mindestens 4 aufeinander folgende Peaks aufgelöst werden (mit Peaks 2 - 5 bezeichnet). Bevorzugte Aluminiumzirconiumsalze mit einem hohen HPLC-Peak 5-Aluminium-Gehalt (auch als "E⁵AZCH" bezeichnet) sind beispielsweise offenbart in US 6436381 und US 6649152.

Weiterhin sind solche aktivierten "E⁵AZCH"-Salze bevorzugt, deren HPLC-Peak 4-zu-Peak 3-Flächenverhältnis von mindestens 0,4, bevorzugt mindestens 0,7, besonders bevorzugt mindestens 0,9, beträgt. Weitere besonders bevorzugte Antitranspirant-Wirkstoffe sind solche Aluminiumzirconiumsalze mit einem hohen HPLC-Peak 5-Aluminium-Gehalt, die zusätzlich mit einem wasserlöslichen Strontiumsalz und/oder mit einem wasserlöslichen Calciumsalz stabilisiert sind. Entsprechende Salze sind z. B. in US 6923952 offenbart.

Die Formulierung der erfindungsgemäßen Mittel in einer bestimmten Darreichungsform, wie beispielsweise einem Antitranspirant-Rollon oder einem Antitranspirantstift, Antitranspirantgel richtet sich bevorzugt nach den Anforderungen des Verwendungszwecks.

Erfindungsgemäße Mittel können in fester, halbfester, flüssiger, disperser, emulgierter, suspendierter, oder gelförmiger Form vorliegen.

In einer besonders bevorzugten Ausführungsform liegen die erfindungsgemäßen Mittel in flüssiger oder in viskoser, fließfähiger Form vor. Die Applikation kann bevorzugt mit einem Rollkugelapplikator erfolgen. Solche Roller weisen eine in einem Kugelbett gelagerte Kugel auf, die durch Bewegung über eine Oberfläche bewegt werden kann. Dabei nimmt die Kugel etwas von dem zu verteilenden Mittel auf und befördert dieses an die zu behandelnde Oberfläche. Die Verpackung für die erfindungsgemäßen Mittel kann undurchsichtig, aber auch transparent oder transluzent sein.

Unter den Begriff der Flüssigkeit fallen im Sinne der Erfindung auch jegliche Festkörperdispersionen in Flüssigkeiten. Erfindungsgemäße Mittel können auch als Pasten, Salben, Lotionen oder Cremes vorliegen. Feste Mittel können beispielsweise als Stift vorliegen.

Die Applikation kann auch mit Substraten erfolgen, die mit einem erfindungsgemäßen Mittel beaufschlagt sind. Besonders bevorzugt sind Feuchttücher, d.h. für den Anwender vorgefertigte, bevorzugt einzeln abgepackte, Feuchttücher, wie sie z. B. aus dem Bereich der Glasreinigung oder aus dem Bereich der feuchten Toilettenpapiere wohlbekannt sind. Solche Feuchttücher, die vorteilhaft auch Konservierungsstoffe enthalten können, sind dann bevorzugt mit einem erfindungsgemäßen Mittel imprägniert oder beaufschlagt, bevorzugt sind sie einzeln verpackt. Sie können z. B. als Deodorant-Tuch eingesetzt werden, was besonders interessant für den Gebrauch unterwegs ist.

Bevorzugte Substratmaterialien sind bevorzugt ausgewählt aus porösen flächigen Tüchern. Sie können aus einem faserigen oder zellulären flexiblen Material bestehen, das ausreichend mechanische Stabilität und gleichzeitig Weichheit zur Anwendung auf der Haut aufweist. Zu diesen Tüchern gehören Tücher aus gewebtem und ungewebtem synthetischen und natürlichen Fasern, Filz, Papier oder Schaumstoff, wie hydrophilem Polyurethanschaum.

Bevorzugt werden hier herkömmliche Tücher aus ungewebtem Material (Vliese) verwendet. Vliese sind im allgemeinen als adhäsiv gebondete faserige Produkte definiert, die eine Matte oder geschichtete Faserstruktur aufweisen, oder solche, die Fasermatten umfassen, bei denen die Fasern zufällig oder in statistischer Anordnung verteilt sind. Die Fasern können natürlich sein, wie Wolle, Seide, Jute, Hanf, Baumwolle, Lein, Sisal oder Ramie; oder synthetisch, wie Rayon, Celluloseester, Cellulose, Polyvinylderivate, Polyolefine, Polyamide oder Polyester. Im Allgemeinen ist jeder Faserdurchmesser bzw. -titer für die vorliegende Erfindung geeignet. Die hier eingesetzten ungewebten Stoffe neigen aufgrund der zufälligen oder statistischen Anordnung von Fasern in dem ungewebten Material, die ausgezeichnete Festigkeit in allen Richtungen verleihen, nicht zum Zerreißen oder Zerfallen. Beispiele für ungewebte Stoffe, die sich als Substrate in der vorliegenden Erfindung eignen, sind beispielsweise aus WO 98/18441 bekannt. Bevorzugte poröse und flächige Reinigungstücher bestehen aus einem oder verschiedenen Fasermaterialien, insbesondere aus Baumwolle, veredelter Baumwolle, Polyamid, Polyester oder Mischungen aus diesen. Bevorzugt weisen die Substrate in Tuchform eine Fläche von 10 bis 400 cm², bevorzugt von 50 bis 300 cm², besonders bevorzugt von 100 bis 200 cm² auf. Die Grammatur des Tuchmaterials beträgt dabei üblicherweise 20 bis 1000 g/m², bevorzugt 30 bis 500 g/m² und insbesondere 50 bis 150 g/m². Erfindungsgemäß bevorzugte deodorierende oder schweißhemmende Substrate können durch Tränken oder Imprägnierung oder auch durch Aufschmelzen eines erfindungsgemäßen Mittels auf ein Substrat erhalten werden.

Die erfindungsgemäßen Mittel, bevorzugt flüssigen Mittel, können auch mehrphasig sein, die Phasen können z.B. horizontal, also übereinander, oder vertikal, also nebeneinander, angeordnet sein. Es kann sich auch um ein disperses System handeln, bei dem z.B. die festen Bestandteile inhomogen in der flüssigen Matrix verteilt sind, so dass ein solches disperses System vor der Anwendung geschüttelt werden sollte.

In einer bevorzugten Ausführungsform liegen die erfindungsgemäßen Mittel als Wasser-in-Öl-Emulsion vor, die Methansulfonsäure enthält. In einer anderen bevorzugten Ausführungsform liegen die erfindungsgemäßen Mittel als Öl-in-Wasser-Emulsion vor, die Methansulfonsäure, bevorzugt in einer Gesamtmenge von 0,05 - 5 Gew.-%, bevorzugt 0,1 - 3 Gew.-%, besonders bevorzugt 0,5 - 2,5 Gew.-%, außerordentlich bevorzugt 1 - 2 Gew.-%, enthält, jeweils bezogen auf das Gesamtgewicht des Mittels.

In einer weiteren bevorzugten Ausführungsform sind die erfindungsgemäßen Mittel dadurch gekennzeichnet, dass sie eine Antitranspirant-Wasser-in-Öl-Emulsion sind, die Methansulfonsäure enthält, bevorzugt in einer Gesamtmenge von 0,05 - 5 Gew.-%, bevorzugt 0,1 - 3 Gew.-%, besonders bevorzugt 0,5 - 2,5 Gew.-%, außerordentlich bevorzugt 1 - 2 Gew.-%, weiterhin 15 - 90 Gew.-%, bevorzugt 25 - 70 Gew.-%, besonders bevorzugt 30 - 50 Gew.-%, außerordentlich bevorzugt 35 - 45 Gew.-%, Wasser, mindestens einen Emulgator sowie mindestens ein kosmetisches Fett oder Öl, wobei sich die Gew.-%-Angaben jeweils auf das Gesamtgewicht des erfindungsgemäßen Mittels beziehen.

In einer weiteren bevorzugten Ausführungsform sind die erfindungsgemäßen Mittel dadurch gekennzeichnet, dass sie eine Antitranspirant-Öl-in-Wasser-Emulsion sind, die Methansulfonsäure enthält, bevorzugt in einer Gesamtmenge von 0,05 - 5 Gew.-%, bevorzugt 0,1 - 3 Gew.-%, besonders bevorzugt 0,5 - 2,5 Gew.-%, außerordentlich bevorzugt 1 - 2 Gew.-%; weiterhin 15 - 90 Gew.-%, bevorzugt 25 - 70 Gew.-%, besonders bevorzugt 30 - 50 Gew.-%, außerordentlich bevorzugt 35 - 45 Gew.-%, Wasser, mindestens einen Emulgator sowie mindestens ein kosmetisches Fett oder Öl, wobei sich die Gew.-%-Angaben jeweils auf das Gesamtgewicht des erfindungsgemäßen Mittels beziehen.

In einer weiteren bevorzugten Ausführungsform sind die erfindungsgemäßen Mittel dadurch gekennzeichnet, dass sie in einer Gesamtmenge von 0,05 - 5 Gew.-%, bevorzugt 0,1 - 3 Gew.-%, besonders bevorzugt 0,5 - 2,5 Gew.-%, außerordentlich bevorzugt 1 - 2 Gew.-%, Methansulfonsäure, weiterhin Wasser in einer Gesamtmenge von 20 - 65 Gew.-%, bevorzugt 25 - 60 Gew.-%, besonders bevorzugt 30 - 55 Gew.-%, außerordentlich bevorzugt 35 - 50 Gew.-%, weiterhin Ethanol in einer Gesamtmenge von 5 - 60 Gew.-%, bevorzugt 10 - 40 Gew.-%, besonders bevorzugt 15 - 35 Gew.-%, außerordentlich bevorzugt 20 - 30 Gew.-%, sowie mindestens eine ein Hydrogel bildende Substanz in einer Gesamtmenge von 0,01 - 2 Gew.-%, bevorzugt 0,1 - 1 Gew.-% bevorzugt 0,2 - 0,7 Gew.-%, außerordentlich bevorzugt 0,3 - 0,5 Gew.-%, enthalten, wobei sich die Gew.-%-Angaben jeweils auf das Gesamtgewicht des erfindungsgemäßen Mittels beziehen; besonders bevorzugt liegen diese erfindungsgemäßen Mittel als Roll-on-Zusammensetzung vor, die bevorzugt in einem Rollkugelapplikator verpackt ist und die besonders bevorzugt eine dynamische Viskosität im Bereich von 300 - 10000 mPas, bevorzugt 800 - 7500 mPas, besonders bevorzugt 1000 - 5000 mPas, aufweist, gemessen mit einem Brookfield-Viskosimeter, Spindel RV 4, 20 s⁻¹, ohne Helipath, bei 20 °C Umgebungstemperatur und 20 °C Probentemperatur.

In einer weiteren bevorzugten Ausführungsform sind die erfindungsgemäßen Mittel dadurch gekennzeichnet, dass sie in einer Gesamtmenge von 0,05 - 5 Gew.-%, bevorzugt 0,1 - 3 Gew.-%, besonders bevorzugt 0,5 - 2,5 Gew.-%, außerordentlich bevorzugt 1 - 2 Gew.-%, Methansulfonsäure, weiterhin Wasser in einer Gesamtmenge von 15 - 90 Gew.-%, bevorzugt 25 - 60 Gew.-%, besonders bevorzugt 30 - 55 Gew.-%, außerordentlich bevorzugt 35 - 50 Gew.-%, weiterhin Ethanol in einer Gesamtmenge von 5 - 50 Gew.-%, bevorzugt 10 - 40 Gew.-%, besonders bevorzugt 15 - 35 Gew.-%, außerordentlich bevorzugt 20 - 30 Gew.-%, sowie mindestens eine ein Hydrogel bildende Substanz in einer Gesamtmenge von 0,01 - 2 Gew.-%, bevorzugt 0,1 - 1 Gew.-% bevorzugt 0,2 - 0,7 Gew.-%, außerordentlich bevorzugt 0,3 - 0,5 Gew.-%, enthalten, wobei sich die Gew.-%-Angaben jeweils auf das Gesamtgewicht des erfindungsgemäßen Mittels beziehen; besonders bevorzugt liegen auch diese erfindungsgemäßen Mittel als Roll-on-Zusammensetzung vor, die bevorzugt in einem Rollkugelapplikator verpackt ist und die besonders bevorzugt eine dynamische Viskosität im Bereich von 300 - 10000 mPas, bevorzugt 800 - 7500 mPas, besonders bevorzugt 1000 - 5000 mPas, aufweist, gemessen mit einem Brookfield-Viskosimeter, Spindel RV 4, 20 s⁻¹, ohne Helipath, bei 20 °C Umgebungstemperatur und 20 °C Probentemperatur.

In einer weiteren bevorzugten Ausführungsform sind die erfindungsgemäßen Mittel dadurch gekennzeichnet, dass sie als Wasser-in-Öl-Emulsion vorliegen und in einer Gesamtmenge von 0,05 - 5 Gew.-%, bevorzugt 0,1 - 3 Gew.-%, besonders bevorzugt 0,5 - 2,5 Gew.-%, außerordentlich bevorzugt 1 - 2 Gew.-%, Methansulfonsäure, weiterhin Wasser in einer Gesamtmenge von 15 - 90 Gew.-%, bevorzugt 25 - 60 Gew.-%, besonders bevorzugt 30 - 55 Gew.-%, außerordentlich bevorzugt 35 - 50 Gew.-%, sowie mindestens eine ein Hydrogel bildende Substanz in einer Gesamtmenge von 0,01 - 2 Gew.-%, bevorzugt 0,1 - 1 Gew.-% bevorzugt 0,2 - 0,7 Gew.-%, außerordentlich bevorzugt 0,3 - 0,5 Gew.-%, enthalten, wobei sich die Gew.-%-Angaben jeweils auf das Gesamtgewicht des erfindungsgemäßen Mittels beziehen; besonders bevorzugt liegen auch diese erfindungsgemäßen Mittel als Roll-on-Zusammensetzung vor, die bevorzugt in einem Rollkugelapplikator verpackt ist und die besonders bevorzugt eine dynamische Viskosität im Bereich von 300 - 10000 mPas, bevorzugt 800 - 7500 mPas, besonders bevorzugt 1000 - 5000 mPas, aufweist, gemessen mit einem Brookfield-Viskosimeter, Spindel RV 4, 20 s⁻¹, ohne Helipath, bei 20 °C Umgebungstemperatur und 20 °C Probentemperatur.

In einer anderen bevorzugten Ausführungsform sind die erfindungsgemäßen Mittel dadurch gekennzeichnet, dass sie als Öl-in-Wasser-Emulsion vorliegen und in einer Gesamtmenge von 0,05 - 5 Gew.-%, bevorzugt 0,1 - 3 Gew.-%, besonders bevorzugt 0,5 - 2,5 Gew.-%, außerordentlich bevorzugt 1 - 2 Gew.-%, Methansulfonsäure, weiterhin Wasser in einer Gesamtmenge von 15 - 90 Gew.-%, bevorzugt 25 - 60 Gew.-%, besonders bevorzugt 30 - 55 Gew.-%, außerordentlich bevorzugt 35 - 50 Gew.-%, weiterhin Ethanol in einer Gesamtmenge von 5 - 60 Gew.-%, bevorzugt 10 - 40 Gew.-%, besonders bevorzugt 15 - 35 Gew.-%, außerordentlich bevorzugt 20 - 30 Gew.-%, sowie mindestens eine ein Hydrogel bildende Substanz in einer Gesamtmenge von 0,01 - 2 Gew.-%, bevorzugt 0,1 - 1 Gew.-% bevorzugt 0,2 - 0,7 Gew.-%, außerordentlich bevorzugt 0,3 - 0,5 Gew.-%, enthalten, wobei sich die Gew.-%-Angaben jeweils auf das Gesamtgewicht des erfindungsgemäßen Mittels beziehen; besonders bevorzugt liegen auch diese erfindungsgemäßen Mittel als Roll-on-Zusammensetzung vor, die bevorzugt in einem Rollkugelapplikator verpackt ist und die besonders bevorzugt eine dynamische Viskosität im Bereich von 300 - 10000 mPas, bevorzugt 800 - 7500 mPas, besonders bevorzugt 1000 - 5000 mPas, aufweist, gemessen mit einem Brookfield-Viskosimeter, Spindel RV 4, 20 s⁻¹, ohne Helipath, bei 20 °C Umgebungstemperatur und 20 °C Probentemperatur.

Erfindungsgemäß bevorzugte Hydrogel bildende Substanzen sind ausgewählt aus Celluloseethern, vor allem Hydroxyalkylcellulosen, insbesondere Hydroxypropylcellulose, Hydroxypropylmethylcellulose, Hydroxyethylcellulose, Carboxymethylcellulose, Cetylhydroxyethylcellulose, Hydroxybutylmethylcellulose, Methylhydroxyethylcellulose, weiterhin Xanthan-Gum, Sclerotium Gum, Succinoglucanen, Polygalactomannanen, insbesondere Guar-Gums und Johannisbrotkernmehl (Locust Bean Gum), insbesondere Guar-Gum und Locust Bean Gum selbst und den nichtionischen Hydroxyalkylguarderivaten und Johannisbrotkernmehl-Derivaten, wie Hydroxypropylguar, Carboxymethylhydroxypropylguar, Hydroxypropylmethylguar, Hydroxyethylguar und Carboxymethylguar, weiterhin Pectinen, Agar, Carragheen (Carrageenan), Traganth, Gummi arabicum, Karayagummi, Taragummi, Gellan, Gelatine, Casein, Propylenglycolalginat, Alginsäuren und deren Salze, insbesondere Natriumalginat, Kaliumalginat und Calciumalginat, weiterhin Polyvinylpyrrolidonen, Polyvinylalkoholen, Polyacrylamiden, weiterhin - wenn auch weniger bevorzugt - physikalisch (z. B. durch Vorverkleisterung) und/oder chemisch modifizierten Stärken, insbesondere hydroxypropylierten Stärkephosphaten und Octenylstärkesuccinaten und deren Aluminium-, Calcium- oder Natriumsalzen, weiterhin - ebenfalls weniger bevorzugt - Acrylsäure-Acrylat-Copolymeren, Acrylsäure-Acrylamid-Copolymeren, Acrylsäure-Vinylpyrrolidon-Copolymeren, Acrylsäure-Vinylformamid-Copolymeren und Polyacrylaten. Besonders bevorzugte Hydrogelbildner sind ausgewählt aus Celluloseethern, vor allem aus Hydroxyalkylcellulosen, insbesondere aus Hydroxypropylcellulose, Hydroxypropylmethylcellulose, Hydroxyethylcellulose, Carboxymethylcellulose, Cetylhydroxyethylcellulose, Hydroxybutylmethylcellulose und Methylhydroxyethylcellulose, sowie Mischungen hiervon. Außerordentlich bevorzugte Hydrogelbildner sind Hydroxyethylcellulose sowie Aluminiumoctenylstärkesuccinat.

In einer weiteren bevorzugten Ausführungsform sind die erfindungsgemäßen Mittel dadurch gekennzeichnet, dass sie Wasser in einer Gesamtmenge von 15 - 90 Gew.-%, bevorzugt 25 - 70 Gew.-%, besonders bevorzugt 30 - 60 Gew.-%, außerordentlich bevorzugt 35 - 50 Gew.-%, weiterhin Ethanol in einer Gesamtmenge von 5 - 50 Gew.-%, bevorzugt 10 - 40 Gew.-%, besonders bevorzugt 15 - 35 Gew.-%, außerordentlich bevorzugt 20 - 30 Gew.-%, sowie Hydroxyethylcellulose in einer Gesamtmenge von 0,01 - 2 Gew.-%, bevorzugt 0,1 - 1 Gew.-% bevorzugt 0,2 - 0,7 Gew.-%, außerordentlich bevorzugt 0,3 - 0,5 Gew.-%, enthalten, wobei sich die Gew.-%-Angaben jeweils auf das Gesamtgewicht des erfindungsgemäßen Mittels beziehen; besonders bevorzugt liegen diese erfindungsgemäßen Mittel als Roll-on-Zusammensetzung vor, die bevorzugt in einem Rollkugelapplikator verpackt ist und die besonders bevorzugt eine dynamische Viskosität im Bereich von 300 - 10000 mPas, bevorzugt 800 - 7500 mPas, besonders bevorzugt 1000 - 5000 mPas, aufweist, gemessen mit einem Brookfield-Viskosimeter, Spindel RV 4, 20 s⁻¹, ohne Helipath, bei 20 °C Umgebungstemperatur und 20 °C Probentemperatur.

In einer weiteren bevorzugten Ausführungsform sind die erfindungsgemäßen Mittel dadurch gekennzeichnet, dass sie in einer Gesamtmenge von 0,05 - 5 Gew.-%, bevorzugt 0,1 - 3 Gew.-%, besonders bevorzugt 0,5 - 2,5 Gew.-%, außerordentlich bevorzugt 1 - 2 Gew.-%, Methansulfonsäure, weiterhin Wasser in einer Gesamtmenge von 15 - 90 Gew.-%, bevorzugt 25 - 70 Gew.-%, besonders bevorzugt 30 - 60 Gew.-%, außerordentlich bevorzugt 35 - 50 Gew.-%, sowie Hydroxyethylcellulose in einer Gesamtmenge von 0,01 - 2 Gew.-%, bevorzugt 0,1 - 1 Gew.-% bevorzugt 0,2 - 0,7 Gew.-%, außerordentlich bevorzugt 0,3 - 0,5 Gew.-%, enthalten, wobei sich die Gew.-%-Angaben jeweils auf das Gesamtgewicht des erfindungsgemäßen Mittels beziehen; besonders bevorzugt liegen auch diese erfindungsgemäßen Mittel als Roll-on-Zusammensetzung vor, die bevorzugt in einem Rollkugelapplikator verpackt ist und die besonders bevorzugt eine dynamische Viskosität im Bereich von 300 - 10000 mPas, bevorzugt 800 - 7500 mPas, besonders bevorzugt 1000 - 5000 mPas, aufweist, gemessen mit einem Brookfield-Viskosimeter, Spindel RV 4, 20 s⁻¹, ohne Helipath, bei 20 °C Umgebungstemperatur und 20 °C Probentemperatur.

In einer weiteren bevorzugten Ausführungsform sind die erfindungsgemäßen Mittel dadurch gekennzeichnet, dass sie als Öl-in-Wasser-Emulsion vorliegen, in einer Gesamtmenge von 0,05 - 5 Gew.-%, bevorzugt 0,1 - 3 Gew.-%, besonders bevorzugt 0,5 - 2,5 Gew.-%, außerordentlich bevorzugt 1 - 2 Gew.-%, Methansulfonsäure und/oder mindestens ein physiologisch verträgliches Salz davon, weiterhin Wasser in einer Gesamtmenge von 15 - 90 Gew.-%, bevorzugt 25 - 60 Gew.-%, besonders bevorzugt 30 - 55 Gew.-%, außerordentlich bevorzugt 35 - 50 Gew.-%, weiterhin Ethanol in einer Gesamtmenge von 5 - 60 Gew.-%, bevorzugt 10 - 40 Gew.-%, besonders bevorzugt 15 - 35 Gew.-%, außerordentlich bevorzugt 20 - 30 Gew.-%, sowie Aluminiumstärkeoctenylsuccinat in einer Gesamtmenge von 0,01 - 2 Gew.-%, bevorzugt 0,1 - 1 Gew.-% bevorzugt 0,2 - 0,7 Gew.-%, außerordentlich bevorzugt 0,3 - 0,5 Gew.-%, enthalten, wobei sich die Gew.-%-Angaben jeweils auf das Gesamtgewicht des erfindungsgemäßen Mittels beziehen; besonders bevorzugt liegen auch diese erfindungsgemäßen Mittel als Roll-on-Zusammensetzung vor, die bevorzugt in einem Rollkugelapplikator verpackt ist und die besonders bevorzugt eine dynamische Viskosität im Bereich von 300 - 10000 mPas, bevorzugt 800 - 7500 mPas, besonders bevorzugt 1000 - 5000 mPas, aufweist, gemessen mit einem Brookfield-Viskosimeter, Spindel RV 4, 20 s⁻¹, ohne Helipath, bei 20 °C Umgebungstemperatur und 20 °C Probentemperatur.

Um die Verfärbungs-inhibierende Wirkung der Methansulfonsäure weiter zu unterstützen, kann es von Vorteil sein, den erfindungsgemäßen Mitteln mindestens einen Chelatbildner, der ausgewählt ist aus Ethylendiamintetraessigsäure (EDTA) und ihren Salze sowie aus Nitrilotriessigsäure (NTA) und Mischungen dieser Substanzen, in einer Gesamtmenge von 0,01 - 2,0 Gew.-%, bevorzugt 0,02 - 1 Gew.-%, besonders bevorzugt 0,05 - 0,3 Gew.-%, zuzusetzen.

Weitere erfindungsgemäß bevorzugte Mittel sind daher dadurch gekennzeichnet, dass zusätzlich mindestens ein Chelatbildner, der ausgewählt ist aus Ethylendiamintetraessigsäure (EDTA) und ihren Salzen sowie aus Nitrilotriessigsäure (NTA) und Mischungen dieser Substanzen, in einer Gesamtmenge von 0,01 - 0,5 Gew.-%, bevorzugt 0,02 - 0,3 Gew.-%, besonders bevorzugt 0,05 - 0,1 Gew.-%, enthalten ist, wobei sich die Gew.-%-Angaben jeweils auf das Gesamtgewicht des Mittels beziehen.

Weitere erfindungsgemäß bevorzugte Zusammensetzungen enthalten optional mindestens einen Deodorant-Wirkstoff in einer Gesamtmenge von 0,0001 - 40 Gew.-%, bevorzugt 0,2 - 20 Gew.-%, besonders bevorzugt 1 - 15 Gew.-%, außerordentlich bevorzugt 1,5 - 5 Gew.-%, wobei sich die Gew.-%-Angaben auf das Gesamtgewicht der Zusammensetzung beziehen.

Ethanol gilt erfindungsgemäß nicht als Deodorant-Wirkstoff, sondern, sofern vorhanden, nur als Bestandteil des Trägers.

In einer bevorzugten Ausführungsform enthalten die erfindungsgemäßen Mittel als deodorierenden Wirkstoff mindestens ein Silbersalz, das bevorzugt ausgewählt ist aus Silbersulfat, Silbernitrat, Silbercitrat, Silberdihydrogencitrat, Silberlactat, Silberacetat, Silbermalat, Silbersuccinat, Silbertartrat, Silbermandelat, Silbersalicylat, Silbergluconat, Silberadipat und Silbergalactarat, sowie aus Mischungen dieser Salze. Außerordentlich bevorzugt sind Silbersulfat, Silbercitrat, Silberdihydrogencitrat und Silberlactat, sowie Mischungen dieser Salze.

Weitere bevorzugte erfindungsgemäße Zusammensetzungen enthalten mindestens ein Silbersalz, das bevorzugt ausgewählt ist aus Silbersulfat, Silbernitrat, Silbercitrat, Silberdihydrogencitrat, Silberlactat, Silberacetat, Silbermalat, Silbersuccinat, Silbertartrat, Silbermandelat, Silbersalicylat, Silbergluconat, Silberadipat und Silbergalactarat, sowie aus Mischungen dieser Salze, in solchen Mengen, dass Silber in einer Gesamtmenge von 1 - 100 ppm, bevorzugt 2 - 50 ppm, besonders bevorzugt 5 - 20 ppm, außerordentlich bevorzugt 7 - 10 ppm, jeweils bezogen auf das Gewicht der Zusammensetzung, enthalten ist. Anhand der Molmassen von Silber (107,87 g/mol) und den jeweiligen Silbersalzen - Silberlactat z. B. hat eine Molmasse von 196,94 g/mol - kann die entsprechend nötige Menge an Silbersalz(en) berechnet werden.

In einer weiteren bevorzugten Ausführungsform enthalten die erfindungsgemäßen Mittel als deodorierenden Wirkstoff mindestens einen aromatischen Alkohol der Struktur (AA-1), 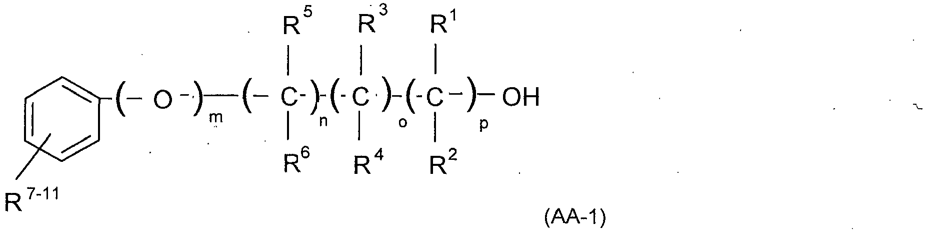 wobei
die Reste R¹ bis R⁶ unabhängig voneinander ein Wasserstoffatom, eine Alkylgruppe mit 1 bis 10 Kohlenstoffatomen, die linear oder verzweigt und substituiert sein können mit OH-Gruppen oder Alkoxy-Gruppen mit 1 bis 5 Kohlenstoffatomen, oder eine Alkenylgruppe mit 2 bis 10 Kohlenstoffatomen, die linear oder verzweigt und substituiert sein können mit OH-Gruppen oder Alkoxy-Gruppen mit 1 bis 5 Kohlenstoffatomen,
die Reste R⁷ bis R¹¹ unabhängig voneinander ein Wasserstoffatom, ein Halogenatom, insbesondere ein Chloratom, oder eine Alkylgruppe mit 1 bis 10 Kohlenstoffatomen, die linear oder verzweigt und substituiert sein können mit OH-Gruppen oder Alkoxy-Gruppen mit 1 bis 5 Kohlenstoffatomen, insbesondere mit einer Methoyxgruppe,
m = 0 oder 1 ist, n, o, p = unabhängig voneinander ganze Zahlen von 0 bis 10 sind, wobei mindestens einer der Werte n, o, p ≠ 0 ist.

Besonders bevorzugte erfindungsgemäße Produkte enthalten mindestens einen Alkohol AA-1, wie vorstehend beschrieben, der ausgewählt ist aus Anisalkohol, 2-Methyl-5-phenyl-pentan-1-ol, 1,1-Dimethyl-3-phenyl-propan-1-ol, Benzylalkohol, 2-Phenylethan-1-ol, 3-Phenylpropan-1-ol, 4-Phenylbutan-1-ol, 5-Phenylpentan-1-ol, 2-Benzylheptan-1-ol, 2,2-Dimethyl-3-phenylpropan-1-ol, 2,2-Dimethyl-3-(3'-methylphenyl)-propan-1-ol, 2-Ethyl-3-phenylpropan-1-ol, 2-Ethyl-3-(3'-methylphenyl)-propan-1-ol, 3-(3'-Chlorphenyl)-2-ethylpropan-1-ol, 3-(2'-Chlorphenyl)-2-ethylpropan-1-ol, 3-(4'-Chlorphenyl)-2-ethylpropan-1-ol, 3-(3',4'-Dichlorphenyl)-2-ethylpropan-1-ol, 2-Ethyl-3-(2'-methylphenyl)-propan-1-ol, 2-Ethyl-3-(4'-methylphenyl)-propan-1-ol, 3-(3',4'-Dimethylphenyl)-2-ethylpropan-1-ol, 2-Ethyl-3-(4'-methoxyphenyl)-propan-1-ol, 3-(3',4'-Dimethoxyphenyl)-2-ethylpropan-1-ol, 2-Allyl-3-phenylpropan-1-ol und 2-n-Pentyl-3-phenylpropan-1-ol sowie Mischungen hiervon. Außerordentlich bevorzugt ist 2-Benzylheptan-1-ol sowie Mischungen von 2-Benzylheptan-1-ol und Phenoxyethanol. Weitere besonders bevorzugte erfindungsgemäße Zusammensetzungen enthalten mindestens einen Alkohol AA-1, wie vorstehend beschrieben, in einer Gesamtmenge von 0,05 - 10 Gew.-%, bevorzugt 0,1 - 5 Gew.-%, besonders bevorzugt 0,2 - 2 Gew.-%, außerordentlich bevorzugt 0,3 - 1,5 Gew.-%, jeweils bezogen auf das Gewicht der Zusammensetzung. Außerordentlich bevorzugte erfindungsgemäße Mittel enthalten 2-Benzylheptan-1-ol in einer Gesamtmenge von 0,05 - 1,5 Gew.-%, bevorzugt 0,1 - 1 Gew.-%, besonders bevorzugt 0,2 - 0,5 Gew.-%, jeweils bezogen auf das Gewicht der Zusammensetzung.

In einer weiteren bevorzugten Ausführungsform enthalten die erfindungsgemäßen Mittel als Deodorant-Wirkstoff mindestens ein 1,2-Alkandiol mit 5 bis 12 C-Atomen, welches durch die Formel HO-CH₂-CH(OH)-(CH₂)ₙ-CH₃ beschrieben wird, in der n für die Zahlen 2, 3, 4, 5, 6, 7, 8 oder 9 steht, sowie Mischungen dieser 1,2-Alkandiole. Erfindungsgemäß besonders bevorzugte 1,2-Alkandiole mit 5 bis 12 C-Atomen sind ausgewählt aus 1,2-Pentandiol, 1,2-Hexandiol, 1,2-Octandiol, 1,2-Decandiol und Mischungen hiervon. Eine ganz besonders bevorzugte erfindungsgemäße Kombination sind Mischungen aus 1,2-Hexandiol und 1,2-Octandiol, vorzugsweise im Gewichtsverhältnis 10:1 bis 1:10, weiter bevorzugt von 5:1 bis 1:5, besonders bevorzugt im Gewichtsverhältnis 1:1.

Bevorzugte erfindungsgemäße Mittel enthalten mindestens ein 1,2-Alkandiol mit 5 bis 12 C-Atomen, das durch die Formel HO-CH₂-CH(OH)-(CH₂)ₙ-CH₃ beschrieben wird, in der n für die Zahlen 2, 3, 4, 5, 6, 7, 8 oder 9 steht, in einer Gesamtmenge von 0,2 - 15 Gew.-%, bevorzugt 0,3 - 10 Gew.-%, besonders bevorzugt 0,4 - 5 Gew.-% und außerordentlich bevorzugt 0,5 - 2 Gew.-%, jeweils bezogen auf das Gewicht der Zusammensetzung. Außerordentlich bevorzugte erfindungsgemäße Mittel enthalten 0,2 - 0,5 Gew.-% 1,2-Hexandiol und 0,2 - 0,5 Gew.-% 1,2-Octandiol, jeweils bezogen auf das Gewicht der Zusammensetzung.

Weitere bevorzugte erfindungsgemäße Mittel sind gekennzeichnet durch einen Gehalt an dem Deodorant-Wirkstoff 3-(2-Ethylhexyloxy)-1,2-propandiol, bevorzugt in einer Gesamtmenge von 0,05 - 5 Gew.-%, bevorzugt 0,1 - 2 Gew.-%, besonders bevorzugt 0,2 - 1,5 Gew.-%, außerordentlich bevorzugt 0,5 - 1,0 Gew.-%, jeweils bezogen auf das Gewicht der Zusammensetzung.

Weitere bevorzugte erfindungsgemäße Mittel sind gekennzeichnet durch einen Gehalt an Tropolon (2-Hydroxy-2,4,6-Cycloheptatrienon), bevorzugt in einer Menge von 0,001 - 0,1 Gew.-%, bezogen auf das Gewicht der Zusammensetzung.

Weitere bevorzugte erfindungsgemäße Mittel sind gekennzeichnet durch einen Gehalt an dem Deodorant-Wirkstoff Triethylcitrat. Triethylcitrat ist ein bekannter Deodorantwirkstoff, der als Enzyminhibitor für Esterasen und Lipasen wirkt und somit zur Breitbandwirkung erfindungsgemäßer Mittel beiträgt. Bevorzugte erfindungsgemäße Mittel enthalten 0,5 - 15 Gew.-%, bevorzugt 3 - 8 Gew.-%, außerordentlich bevorzugt 4 - 6 Gew.-%, jeweils bezogen auf das Gewicht der Zusammensetzung.

Apokriner Schweiß stellt eine komplexe Mischung dar, die unter anderem Sebum und andere Fette sowie Steroide enthält. Steroide selbst sind nicht wasserlöslich. Um mit den Körperflüssigkeiten abtransportiert werden zu können, liegen sie normalerweise als Sulfat oder als Glucuronid vor. Auf der Haut erfolgt die Spaltung dieser Steroidester in die flüchtigen freien Steroide durch hydrolytische Enzyme der Hautbakterien, insbesondere der coryneformen Bakterien. Prinzipiell sind dazu alle bakteriellen Exoesterasen in der Lage, besonders aber die Enzyme Arylsulfatase und beta-Glucuronidase. Verbindungen, die Arylsulfatase oder beta-Glucuronidase inhibieren, stellen daher erfindungsgemäß bevorzugte Deodorantwirkstoffe dar.

Die Entwicklung der kurz- und mittelkettigen Fettsäuren, die wesentlich zum Körpergeruch beitragen, beginnt mit der Spaltung von Hautlipiden zu verzweigten langkettigen Fettsäuren. Die Spaltung der Hautlipide, die überwiegend als Glycerinester vorliegen, erfolgt im Wesentlichen durch Propionibacterium, Corynebacterium A und Staphylococcus-Spezies (A.G. James et al., Generation and Turnover of Volatile Fatty Acids by Axillary Bacteria, 22nd IFSCC Congress, Edinburgh, 2002, Poster 108). A.G. James et al. offenbaren weiterhin, dass aus den langkettigen verzweigten Fettsäuren durch die hydrolytischen Enzyme von einem bestimmten Corynebacterium, das A.G. James et al. als Corynebacterium A bezeichnen, sowohl kurzkettige C₂ - C₅-Fettsäuren als auch mittelkettige C₆ - C₁₂-Fettsäuren gebildet werden, die hauptsächlich für den axillaren Körpergeruch verantwortlich sind. Prinzipiell sind alle bakteriellen Exoesterasen zu dieser Lipidspaltung fähig, besonders aber das Enzym Lipase. Verbindungen, die Lipase inhibieren, stellen daher ebenfalls erfindungsgemäß bevorzugte Deodorantwirkstoffe dar.

Eine weitere Verbindungsklasse, die ebenfalls bei der bakteriellen Zersetzung der Schweißbestandteile entsteht und zum Körpergeruch beiträgt, sind gesättigte und ungesättigte Aldehyde, vor allem solche mit einer Kettenlänge von C₆ - C₁₂, insbesondere Hexanal, Heptanal, Octenal und Nonenal. Diese entstehen durch beta-Spaltung aus den Hydroperoxiden, die unter Einwirkung der 5-Lipoxigenase auf ungesättigte Fettsäuren gebildet werden. Verbindungen, die das Enzym 5-Lipoxigenase inhibieren, stellen daher ebenfalls erfindungsgemäß bevorzugte Deodorantwirkstoffe dar.

Weiterhin ist bekannt, dass stark übel riechende Komponenten des menschlichen Körpergeruchs und Mundgeruchs insbesondere durch enzymatische Reaktion freigesetzte flüchtige Schwefelverbindungen, so genannte "volatile sulfur compounds" (VSC), darstellen. Schwefelhaltige Verbindungen kommen als wasserlösliche Aminosäurekonjugate mit dem Schweiß auf die menschliche Haut. Dort werden sie von Hautbakterien (vor allem von Staphylokokken und Corynebakterien) durch enzymatische Reaktion freigesetzt. Ein Enzym, das bei der Freisetzung von VSCs eine besondere Rolle spielt, ist die Cystathionin-beta-Lyase. Dieses Enzym spaltet VSCs von den Aminosäuren ab und ist somit eine wichtige Ursache bei der Entstehung von Körpergeruch. Verbindungen, die das Enzym Cystathionin-beta-Lyase inhibieren, stellen daher ebenfalls erfindungsgemäß bevorzugte Deodorantwirkstoffe dar.

Weitere bevorzugte erfindungsgemäße Mittel sind gekennzeichnet durch einen Gehalt an mindestens einer Verbindung, die ein Inhibitor des Enzyms Arylsulfatase ist. Erfindungsgemäß bevorzugte Deodorantwirkstoffe, die als Arylsulfatase-Inhibitor wirken, sind solche, wie sie in US 5643559, US 5676937, WO2001/099376 A2, EP 1430879 A1 und DE 10216368 A1 offenbart sind. Weitere besonders bevorzugte erfindungsgemäße Mittel sind gekennzeichnet durch einen Gehalt an mindestens einer Verbindung, die ein Inhibitor des Enzyms Arylsulfatase ist, in einer Gesamtmenge von 0,001 - 10 Gew.-%, bevorzugt 0,01 - 5 Gew.-%, besonders bevorzugt 0,1 - 2,5 Gew.-%, jeweils bezogen auf das Gewicht der Zusammensetzung.

Weitere bevorzugte erfindungsgemäße Mittel sind gekennzeichnet durch einen Gehalt an mindestens einer Verbindung, die ein Inhibitor des Enzyms beta-Glucuronidase ist. Erfindungsgemäß bevorzugte Deodorantwirkstoffe, die als beta-Glucuronidase-Inhibitor wirken, sind solche, wie sie in WO2003/039505 A2 offenbart sind. Weitere besonders bevorzugte erfindungsgemäße Mittel sind gekennzeichnet durch einen Gehalt an mindestens einer Verbindung, die ein Inhibitor des Enzyms beta-Glucuronidase ist, in einer Gesamtmenge von 0,001 - 10 Gew.-%, bevorzugt 0,01 - 5 Gew.-%, besonders bevorzugt 0,1 - 2,5 Gew.-%, jeweils bezogen auf das Gewicht der Zusammensetzung.

Weitere bevorzugte erfindungsgemäße Mittel sind gekennzeichnet durch einen Gehalt an mindestens einer Verbindung, die ein Inhibitor des Enzyms Lipase ist. Erfindungsgemäß bevorzugte Deodorantwirkstoffe, die als Lipase-Inhibitor wirken, sind ausgewählt aus denen, die in EP 1428520 A2 offenbart sind, weiterhin ausgewählt aus den Aminomethylenmalonsäure-Derivaten gemäß DE 3018132 A1, den Ethylenoxid-Propylenoxid-Copolymeren gemäß GB 2335596 A1 und den Salzen der Phytinsäure gemäß EP 650 720 A1. Weitere besonders bevorzugte erfindungsgemäße Mittel sind gekennzeichnet durch einen Gehalt an mindestens einer Verbindung, die ein Inhibitor des Enzyms Lipase ist, in einer Gesamtmenge von 0,001 - 10 Gew.-%, bevorzugt 0,01 - 5 Gew.-%, besonders bevorzugt 0,1 - 2,5 Gew.-%, jeweils bezogen auf das Gewicht der Zusammensetzung.

Weitere besonders bevorzugte erfindungsgemäße Mittel sind gekennzeichnet durch einen Gehalt an mindestens einer Verbindung, die ein Inhibitor der 5-Lipoxigenase ist. Erfindungsgemäß bevorzugte Deodorantwirkstoffe, die als 5-Lipoxigenase-Inhibitor wirken, sind in EP 1428519 A2 offenbart. Weitere besonders bevorzugte erfindungsgemäße Mittel sind gekennzeichnet durch einen Gehalt an mindestens einer Verbindung, die ein Inhibitor des Enzyms 5-Lipoxigenase ist, in einer Gesamtmenge von 0,001 - 10 Gew.-%, bevorzugt 0,01 - 5 Gew.-%, besonders bevorzugt 0,1 - 2,5 Gew.-%, jeweils bezogen auf das Gewicht der Zusammensetzung.

Weitere besonders bevorzugte erfindungsgemäße Mittel sind gekennzeichnet durch einen Gehalt an mindestens einer Verbindung, die ein Inhibitor des Enzyms Cystathionin-beta-Lyase ist. Erfindungsgemäß bevorzugte Deodorantwirkstoffe, die als Inhibitor der Cystathionin-beta-Lyase wirken, sind ausgewählt aus denen, die in EP 495918 B1, WO 2006/079934, DE 102010000746 A1, WO2010/031657 A1 und WO2010/046291 A1 offenbart sind. Weitere besonders bevorzugte erfindungsgemäße Mittel sind gekennzeichnet durch einen Gehalt an mindestens einer Verbindung, die ein Inhibitor des Enzyms Cystathionin-beta-Lyase ist, in einer Gesamtmenge von 0,001 - 10 Gew.-%, bevorzugt 0,01 - 5 Gew.-%, besonders bevorzugt 0,1 - 2,5 Gew.-%, jeweils bezogen auf das Gewicht der Zusammensetzung.

Weitere besonders bevorzugte erfindungsgemäße Mittel sind gekennzeichnet durch einen Gehalt an mindestens einem kationischen Phospholipid der Formel KPL, 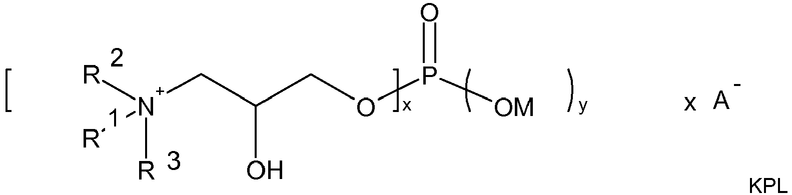 in der R¹ eine Alkyl-, Alkenyl- oder Hydroxyalkylgruppe mit 8 bis 22 C-Atomen oder eine Acylaminoalkylgruppe der Formel R⁵CONH(CₘH₂ₘ)- ist, worin R⁵CO eine lineare Acylgruppe mit 8 bis 22 C-Atomen und m = 2 oder 3 ist, R² und R³ Alkylgruppen mit 1 bis 4 C-Atomen oder Hydroxyalkylgruppen mit 2 bis 4 C- Atomen oder Carboxyalkylgruppen der Formel -(CH₂)_{z}-COOM sind, worin z einen Wert von 1 bis 3 hat und M Wasserstoff oder ein Alkalimetallkation ist, x einen Wert von 1 bis 3 und y einen Wert von (3 - x) hat, M Wasserstoff oder ein Alkalimetallkation ist und A⁻ ein Anion ist.

Bevorzugte Alkylgruppen mit 8 bis 22 C-Atomen sind ausgewählt aus einer n-Octyl-, n-Nonyl-, n-Decyl-, n-Undecyl-, Lauryl-, n-Tridecanyl-, Myristyl-, n-Pentadecanyl-, Cetyl-, Palmityl-, Stearyl-, Elaidyl-, Arachidyl-, Behenyl- und einer Cocyl-Gruppe. Eine repräsentative Cocyl-Gruppe besteht, bezogen auf ihr Gesamtgewicht, aus 4 - 9 Gew.-% n-Octyl-, 4 - 9 Gew.-% n-Decyl-, 45 - 55 Gew.-% Lauryl-, 15 - 21 Gew.-% Myristyl-, 8 - 13 Gew.-% Palmityl- und 7 - 14 Gew.-% Stearyl-Gruppen. Bevorzugte Alkenylgruppen mit 8 bis 22 C-Atomen sind ausgewählt aus einer Linoleyl-Gruppe ((9Z,12Z)-Octadeca-9,12-dien-1-yl) und einer Linolenyl-Gruppe ((9Z,12Z,15Z)-Octadeca-9,12,15-trien-1-yl). Eine bevorzugte Hydroxyalkylgruppe mit 8 bis 22 C-Atomen ist ausgewählt aus einer 12-Hydroxystearylgruppe.

Besonders bevorzugte kationische Phospholipide der Formel KPL sind solche, bei denen R¹ eine Acylaminoalkylgruppe der Formel R⁵CONH(CₘH₂ₘ)- ist, worin R⁵CO eine lineare Acylgruppe mit 8 bis 22 C-Atomen darstellt und m = 3 ist.

Bevorzugte lineare Acylgruppen R⁵CO mit 8 bis 22 C-Atomen sind ausgewählt aus einer n-Octanoyl-, n-Nonanoyl-, n-Decanoyl-, n-Undecanoyl-, Lauroyl-, n-Tridecanoyl-, Myristoyl-, n-Pentadecanoyl-, Cetoyl-, Palmitoyl-, Stearoyl-, Elaidoyl-, Arachidoyl-, Behenoyl- und einer Cocoyl-Gruppe. Eine repräsentative Cocoyl-Gruppe besteht, bezogen auf ihr Gesamtgewicht, aus 4 - 9 Gew.-% n-Octanoyl-, 4 - 9 Gew.-% n-Decanoyl-, 45 - 55 Gew.-% Lauroyl-, 15 - 21 Gew.-% Myristoyl-, 8 - 13 Gew.-% Palmitoyl- und 7 - 14 Gew.-% Stearoyl-Gruppen. Besonders bevorzugte lineare Acylgruppen R⁵CO sind ausgewählt aus einer Cocoyl-Gruppe, einer Lauroyl-Gruppe (n-C₁₁H₂₃CO), einer Myristoyl-Gruppe (n-C₁₃H₂₇CO) und einer Linoleoyl-Gruppe ((9Z,12Z)-Octadeca-9,12-dien-1-oyl). Außerordentlich bevorzugte lineare Acylgruppen R⁵CO sind ausgewählt aus einer Cocoyl-Gruppe, einer Lauroyl-Gruppe (n-C₁₁H₂₃CO) und einer Myristoyl-Gruppe (n-C₁₃H₂₇CO).

Bevorzugte Alkylgruppen mit 1 bis 4 C-Atomen sind eine Methyl-, Ethyl-, n-Propyl, 1-Methylethyl-, n-Butyl, 2-Methylpropyl- und eine tert.-Butyl-Gruppe. Besonders bevorzugt ist die Methylgruppe. Bevorzugte Hydroxyalkylgruppen mit 2 - 4 C-Atomen sind eine 2-Hydroxyethyl- und eine 1-Hydroxyethylgruppe.

Bevorzugte Carboxyalkylgruppen der Formel -(CH₂)_{z}-COOM mit z = 1 bis 3 sind eine Carboxymethyl-, eine Carboxyethyl und eine Carboxy-n-propyl-Gruppe.

Bevorzugte Alkalimetallkationen sind ausgewählt aus Natrium- und Kalium-Kationen; Na⁺ ist besonders bevorzugt. Bevorzugte Anionen sind ausgewählt aus Sulfat, Chlorid, Phosphat, Nitrat, Hydrogencarbonat und Acetat, wobei ein Chloridanion besonders bevorzugt ist.

Bevorzugte erfindungsgemäße Mittel enthalten als deodorierenden Wirkstoff ein kationisches Phospholipid der Formel KPL, 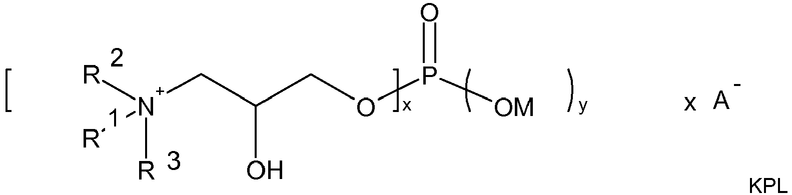 in der R¹ eine Acylaminoalkylgruppe der Formel R⁵CONH(CₘH₂ₘ)- ist, worin R⁵CO ausgewählt ist aus einer Cocoylgruppe, einer Lauroylgruppe, einer Myristoylgruppe und einer Linoleoyl-Gruppe und m = 3 ist, R² und R³ Methylgruppen sind, x = 2, y = 1, M ein Natriumion und A⁻ ein Chloridion sind. Bevorzugt ist mindestens ein kationisches Phospholipid der Formel KPL mit den vorstehend genannten Merkmalen in einer Gesamtmenge von 0,05 - 2 Gew.-%, bevorzugt 0,1 - 1 Gew.-%, besonders bevorzugt 0,15 - 0,4 Gew.-%, enthalten, jeweils bezogen auf das Gewicht des Mittels.

Besonders bevorzugte erfindungsgemäße Mittel enthalten ein kationisches Phospholipid der Formel KPL, 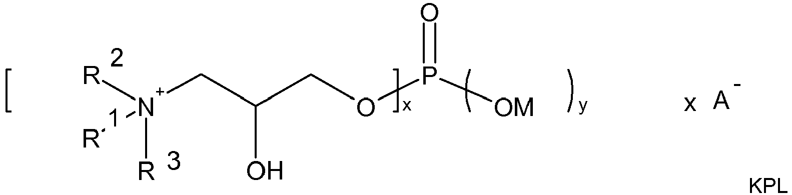 in der R¹ eine Cocoylaminopropylgruppe (auch als Cocamidopropylgruppe bezeichnet) ist, R² und R³ Methylgruppen sind, x = 2, y = 1, M ein Natriumion und A⁻ ein Chloridion sind und die unter der INCI-Bezeichnung Cocamidopropyl PG-Dimonium Chloride Phosphate erhältlich ist, in einer Gesamtmenge von 0,05 - 2 Gew.-%, bevorzugt 0,1 - 1 Gew.-%, besonders bevorzugt 0,15 - 0,4 Gew.-%, jeweils bezogen auf das Gewicht des Mittels.

Weitere besonders bevorzugte erfindungsgemäße Mittel enthalten ein kationisches Phospholipid der Formel KPL, 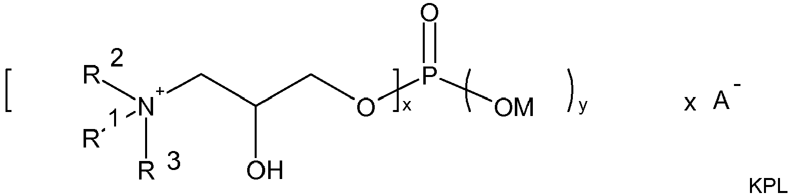 in der R¹ eine Myristoylaminopropylgruppe ist, R² und R³ Methylgruppen sind, x = 2, y = 1, M ein Natriumion und A⁻ ein Chloridion sind und die unter der INCI-Bezeichnung Myristamidopropyl PG-Dimonium Chloride Phosphate erhältlich ist, in einer Gesamtmenge von 0,05 - 2 Gew.-%, bevorzugt 0,1 - 1 Gew.-%, besonders bevorzugt 0,15 - 0,4 Gew.-%, jeweils bezogen auf das Gewicht des Mittels.

Weitere besonders bevorzugte erfindungsgemäße Mittel enthalten ein kationisches Phospholipid der Formel KPL, 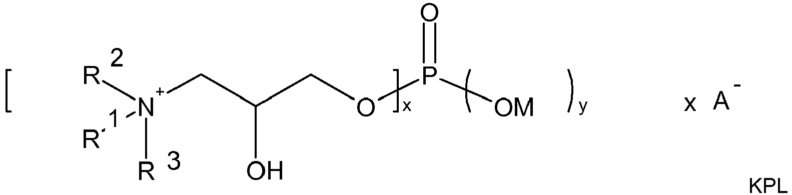 in der R¹ eine Lauroylaminopropylgruppe ist, R² und R³ Methylgruppen sind, x = 2, y = 1, M ein Natriumion und A⁻ ein Chloridion sind, in einer Gesamtmenge von 0,05 - 2 Gew.-%, bevorzugt 0,1 - 1 Gew.-%, besonders bevorzugt 0,15 - 0,4 Gew.-%, jeweils bezogen auf das Gewicht des Mittels.

Weitere erfindungsgemäß bevorzugte Deodorant-Wirkstoffe sind Geruchsabsorber, desodorierend wirkende Ionenaustauscher, keimhemmende Mittel, präbiotisch wirksame Komponenten sowie Enzyminhibitoren oder, besonders bevorzugt, Kombinationen der genannten Wirkstoffe.

Silicate dienen als Geruchsabsorber, die auch gleichzeitig die rheologischen Eigenschaften der erfindungsgemäßen Zusammensetzung vorteilhaft unterstützen. Zu den erfindungsgemäß besonders bevorzugten Silicaten zählen vor allem Schichtsilicate und unter diesen insbesondere Montmorillonit, Kaolinit, Ilit, Beidellit, Nontronit, Saponit, Hectorit, Bentonit, Smectit und Talkum. Weitere bevorzugte Geruchsabsorber sind beispielsweise Zeolithe, Zinkricinoleat, Cyclodextrine, bestimmte Metalloxide, wie z. B. Aluminiumoxid, sowie Chlorophyll. Sie sind bevorzugt in einer Gesamtmenge von 0,1 - 10 Gew.-%, besonders bevorzugt 0,5 - 7 Gew.-% und außerordentlich bevorzugt 1 - 5 Gew.-%, jeweils bezogen auf die Gesamtzusammensetzung, enthalten.

Unter keimhemmenden oder antimikrobiellen Wirkstoffen werden erfindungsgemäß solche Wirkstoffe verstanden, die die Zahl der an der Geruchsbildung beteiligten Hautkeime reduzieren bzw. deren Wachstum hemmen. Zu diesen Keimen zählen unter anderem verschiedene Spezies aus der Gruppe der Staphylokokken, der Gruppe der Corynebakterien, Anaerokokken und Mikrokokken.

Als keimhemmende oder antimikrobielle Wirkstoffe erfindungsgemäß bevorzugt sind insbesondere Organohalogenverbindungen sowie -halogenide, quartäre Ammoniumverbindungen, eine Reihe von Pflanzenextrakten und Zinkverbindungen. Hierzu zählen u. a. Triclosan, Chlorhexidin und Chlorhexidingluconat, 3,4,4-Trichlorcarbanilid, Bromchlorophen, Dichlorophen, Chlorothymol, Chloroxylenol, Hexachlorophen, Dichloro-m-xylenol, Dequaliniumchlorid, Domiphenbromid, Ammoniumphenolsulfonat, Benzalkoniumhalogenide, Benzalkoniumcetylphosphat, Benzalkoniumsaccharinate, Benzethoniumchlorid, Cetylpyridiniumchlorid, Laurylpyridiniumchlorid, Laurylisoquinoliniumbromid, Methylbenzethoniumchlorid. Weiterhin sind Phenol, Phenoxyethanol, Dinatriumdihydroxyethylsulfosuccinylundecylenat, Natriumbicarbonat, Zinklactat, Natriumphenolsulfonat und Zinkphenolsulfonat, Ketoglutarsäure, Terpenalkohole wie z. B. Farnesol, Chlorophyllin-Kupfer-Komplexe, α-Monoalkylglycerinether mit einem verzweigten oder linearen gesättigten oder ungesättigten, gegebenenfalls hydroxylierten C₆ - C₂₂-Alkylrest, besonders bevorzugt α-(2-Ethylhexyl)glycerinether, im Handel erhältlich als Sensiva® SC 50 (ex Schülke & Mayr) sowie Carbonsäureester des Mono-, Di- und Triglycerins (z. B. Glycerinmonolaurat, Diglycerinmonocaprinat) bevorzugte Deodorant-Wirkstoffe.

Bevorzugte erfindungsgemäße Antitranspirant-Mittel enthalten mindestens einen Deodorant-Wirkstoff, der ausgewählt ist aus Silbersalzen, aromatischen Alkoholen der Struktur AA-1 mit den vorstehend genannten Substituenten, 1,2-Alkandiolen mit 5 bis 12 Kohlenstoffatomen, alpha-(2-Ethylhexyl)glycerinether (3-(2-Ethylhexlyoxy)-1,2-propandiol), Tropolon, Triethylcitrat, kationischen Phospholipiden der Formel KPL mit den vorstehend genannten Substituenten, sowie Mischungen hiervon.

Weitere bevorzugte erfindungsgemäße Antitranspirant-Zusammensetzungen sind dadurch gekennzeichnet, dass der mindestens eine Deodorant-Wirkstoff in einer Gesamtmenge von 0,0001 - 20 Gew.-%, bevorzugt 1 - 15 Gew.-%, besonders bevorzugt 1,5 - 5 Gew.-%, enthalten ist, wobei sich die Gew.-%-Angaben auf das Gesamtgewicht der Zusammensetzung beziehen.

Weitere bevorzugte Ausführungsformen der Erfindung sind dadurch gekennzeichnet, dass das erfindungsgemäße kosmetische Mittel in Stiftform, flüssige oder gelförmige Roll-on-Applikation, Creme, Lotion, Lösung, Gel oder auf einem Substrat aufgebracht vorliegt.

Bevorzugte erfindungsgemäße Zusammensetzungen, die als Emulsion, Suspension, Stift oder Gel vorliegen, enthalten bevorzugt weiterhin mindestens ein unter Normalbedingungen flüssiges kosmetisches Öl, das kein Riechstoff und kein ätherisches Öl ist.

Das kosmetische Öl ist unter Normalbedingungen flüssig. Unter ätherischen Ölen werden erfindungsgemäß Gemische aus flüchtigen Komponenten verstanden, die durch Wasserdampfdestillation aus pflanzlichen Rohstoffen hergestellt werden, wie z. B. Citrusöle.

Die Gesamtmenge an unter Normalbedingungen flüssigen kosmetischen Ölen, die kein Riechstoff und kein ätherisches Öl sind, beträgt in erfindungsgemäß bevorzugten Zusammensetzungen 1 - 50 Gew.-%, bevorzugt 1,5 - 20 Gew.-%, besonders bevorzugt 2 - 10 Gew.-%, außerordentlich bevorzugt 3 - 6 Gew.-%, wobei sich die Mengenangaben auf das Gewicht der Zusammensetzung beziehen.

Bei den kosmetischen Ölen unterscheidet man flüchtige und nicht-flüchtige Öle. Unter nicht-flüchtigen Ölen versteht man solche Öle, die bei 20 °C und einem Umgebungsdruck von 1013 hPa einen Dampfdruck von weniger als 2,66 Pa (0,02 mm Hg) aufweisen. Unter flüchtigen Ölen versteht man solche Öle, die bei 20 °C und einem Umgebungsdruck von 1013 hPa einen Dampfdruck von 2,66 Pa - 40000 Pa (0,02 mm - 300 mm Hg), bevorzugt 10 - 12000 Pa (0,1 - 90 mm Hg), besonders bevorzugt 13 - 3000 Pa, außerordentlich bevorzugt 15 - 500 Pa, aufweisen.

Flüchtige kosmetische Öle sind üblicherweise unter cyclischen Siliconölen mit der INCI-Bezeichnung Cyclomethicone ausgewählt. Unter der INCI-Bezeichnung Cyclomethicone werden insbesondere Cyclotrisiloxan (Hexamethylcyclotrisiloxan), Cyclotetrasiloxan (Octamethylcyclotetrasiloxan), Cyclopentasiloxan (Decamethylcyclopentasiloxan) und Cyclohexasiloxan (Dodecamethylcyclohexasiloxan) verstanden. Diese Öle weisen bei 20°C einen Dampfdruck von ca. 13 - 15 Pa auf.

Cyclomethicone sind im Stand der Technik als gut geeignete Öle für kosmetische Zusammensetzungen, insbesondere für deodorierende Zusammensetzungen, wie Stifte, bekannt. Aufgrund ihrer Persistenz in der Umwelt kann es aber erfindungsgemäß bevorzugt sein, auf den Einsatz von Cyclomethiconen zu verzichten. In einer speziell bevorzugten Ausführungsform enthalten die erfindungsgemäßen und erfindungsgemäß verwendeten Zusammensetzungen 0 bis weniger als 1 Gew.-%, bevorzugt maximal 0,1 Gew.-%, Cyclomethicone, bezogen auf das Gewicht der Zusammensetzung.

Ein erfindungsgemäß bevorzugter Cyclomethicone-Ersatzstoff ist eine Mischung aus C₁₃-C₁₆-Isoparaffinen, C₁₂ - C₁₄-Isoparaffinen und C₁₃ - C₁₅-Alkanen, deren Viskosität bei 25°C im Bereich von 2 - 6 mPas liegt und die einen Dampfdruck bei 20°C im Bereich von 10 - 150 Pa, bevorzugt 100 - 150 Pa, aufweist. Eine solche Mischung ist z. B. unter der Bezeichnung SiClone SR-5 von der Firma Presperse Inc. erhältlich.

Weitere bevorzugte flüchtige Siliconöle sind ausgewählt aus flüchtigen linearen Siliconölen, insbesondere flüchtigen linearen Siliconölen mit 2 - 10 Siloxaneinheiten, wie Hexamethyldisiloxan (L₂), Octamethyltrisiloxan (L₃), Decamethyltetrasiloxan (L₄), wie sie z. B. in den Handelsprodukten DC 2-1184, Dow Corning® 200 (0,65 cSt) und Dow Corning® 200 (1,5 cSt) von Dow Corning enthalten sind, und niedermolekulares Phenyl Trimethicone mit einem Dampfdruck bei 20°C von etwa 2000 Pa, wie es z. B. von GE Bayer Silicones/Momentive unter dem Namen Baysilone Fluid PD 5 erhältlich ist.

Bevorzugte erfindungsgemäße Antitranspirant-Zusammensetzungen enthalten wegen des trockeneren Hautgefühls und der schnelleren Wirkstofffreisetzung mindestens ein flüchtiges Siliconöl, das cyclisch oder linear sein kann.

Weitere bevorzugte erfindungsgemäße Zusammensetzungen enthalten wegen des trockeneren Hautgefühls und der schnelleren Freisetzung des Antitranspirant-Wirkstoffs mindestens ein flüchtiges Nichtsiliconöl. Bevorzugte flüchtige Nichtsiliconöle sind ausgewählt aus C₈-C₁₆-Isoparaffinen, insbesondere aus Isononan, Isodecan, Isoundecan, Isododecan, Isotridecan, Isotetradecan, Isopentadecan und Isohexadecan, sowie Mischungen hiervon. Bevorzugt sind C₁₀-C₁₃-Isoparaffin-Mischungen, insbesondere solche mit einem Dampfdruck bei 20°C von 10 - 400 Pa, bevorzugt 13 - 100 Pa.

Dieses mindestens eine C₈-C₁₆-Isoparaffin ist bevorzugt in einer Gesamtmenge von 1 - 50 Gew.-%, bevorzugt 1,5 - 20 Gew.-%, besonders bevorzugt 2 - 10 Gew.-%, außerordentlich bevorzugt 3 - 6 Gew.-%, enthalten, jeweils bezogen auf das Gesamtgewicht der Zusammensetzung.

Weitere erfindungsgemäß bevorzugte Zusammensetzungen enthalten mindestens ein nichtflüchtiges kosmetisches Öl, ausgewählt aus nichtflüchtigen Siliconölen und nichtflüchtigen Nichtsiliconölen. Rückstände von in der Zusammensetzung unlöslichen Bestandteilen, wie Talkum, aber auch die auf der Haut angetrockneten Antitranspirantwirkstoffe (= schweißhemmende Aluminiumsalze), können erfolgreich mit einem nichtflüchtigen Öl maskiert werden. Außerdem können mit einem Gemisch aus verschiedenen Ölen, insbesondere aus nichtflüchtigem und flüchtigem Öl, Parameter wie Hautgefühl, Sichtbarkeit des Rückstands und Stabilität der erfindungsgemäßen Zusammensetzung feinreguliert und besser an die Bedürfnisse der Verbraucher angepasst werden.

Erfindungsgemäß bevorzugte Mittel sind dadurch gekennzeichnet, dass das kosmetische Öl, das kein Riechstoff und kein ätherisches Öl ist, mindestens ein flüchtiges Öl mit einem Dampfdruck von 10 - 3000 Pa bei 20°C, das kein Riechstoff und kein ätherisches Öl ist, in einer Gesamtmenge von 10 - 100 Gew.-%, besonders bevorzugt 30 - 80 Gew.-%, jeweils bezogen auf das Gesamtgewicht der kosmetischen Öle, umfasst.

Selbstverständlich ist es ebenfalls möglich, erfindungsgemäße Mittel mit einem geringen Anteil an flüchtigen Ölen - das heißt, mit 0,5 - 15 Gew.-% an flüchtigen Ölen, bezogen auf das Gesamtgewicht des Mittels - oder sogar ohne flüchtige Öle zu formulieren.

Erfindungsgemäß besonders bevorzugte Öle sind Ester der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können. Hierzu sei angemerkt, dass einige Ester von linearen oder verzweigten C₁-C₂₂-Alkanolen oder C₁₄-C₂₂-Alkenolen und einige Triester des Glycerins mit linearen oder verzweigten C₂-C₂₂-Carbonsäuren, die gesättigt oder ungesättigt sein können, unter Normalbedingungen fest sind, wie beispielsweise Cetylstearat oder Glycerintristearat (= Stearin). Diese unter Normalbedingungen festen Ester stellen erfindungsgemäß keine kosmetischen Öle dar, da sie nicht die Bedingung "unter Normalbedingungen flüssig" erfüllen. Die Zuordnung, ob ein derartiger Ester unter Normalbedingungen flüssig oder fest ist, liegt im Rahmen des Allgemeinwissens des Fachmanns.

Bevorzugt sind Ester der linearen oder verzweigten gesättigten Fettalkohole mit 2 - 5 Kohlenstoffatomen mit linearen oder verzweigten gesättigten oder ungesättigten Fettsäuren mit 3 - 18 Kohlenstoffatomen, die hydroxyliert sein können. Bevorzugte Beispiele hierfür sind Isopropylpalmitat, Isopropylstearat, Isopropylmyristat, 2-Hexyldecylstearat, 2-Hexyldecyllaurat, Isodecylneopentanoat, Isononylisononanoat, 2-Ethylhexylpalmitat und 2-Ethylhexylstearat. Ebenfalls bevorzugt sind Isopropylisostearat, Isopropyloleat, Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Diisotridecylacetat, n-Butylstearat, n-Hexyllaurat, n-Decyloleat, Oleyloleat, Oleylerucat, Erucyloleat, Erucylerucat, Ethylenglycoldioleat, Ethylenglycoldipalmitat, n-Hexyllaurat, n-Decyloleat, Oleyloleat, Oleylerucat, Erucyloleat, C₁₂-C₁₅-Alkyllactat und Di-C₁₂-C₁₃-Alkylmalat sowie die Benzoesäureester von linearen oder verzweigten C₈₋₂₂-Alkanolen. Besonders bevorzugt sind Benzoesäure-C₁₂-C₁₅-Alkylester, z. B. erhältlich als Handelsprodukt Finsolv® TN (C₁₂-C₁₅-Alkylbenzoat), sowie Benzoesäureisostearylester, z. B. erhältlich als Finsolv® SB, 2-Ethylhexylbenzoat, z. B. erhältlich als Finsolv® EB, und Benzoesäure-2-octyldodecylester, z. B. erhältlich als Finsolv® BOD.

Weitere erfindungsgemäß bevorzugte Ölkomponenten sind ausgewählt aus den C₈-C₂₂-Fettalkoholestern einwertiger oder mehrwertiger C₂-C₇-Hydroxycarbonsäuren, insbesondere die Ester der Glycolsäure, Milchsäure, Äpfelsäure, Weinsäure, Citronensäure und Salicylsäure. Solche Ester auf Basis von linearen C_{14/15}-Alkanolen, z. B. C₁₂₋C₁₅-Alkyllactat, und von in 2-Position verzweigten C_{12/13}-Alkanolen sind unter dem Handelsnamen Cosmacol® von der Firma Nordmann, Rassmann GmbH & Co, Hamburg, zu beziehen, insbesondere die Handelsprodukte Cosmacol® ESI, Cosmacol® EMI und Cosmacol® ETI.

Als besonders vorteilhaft, z. B. für die Wirkstofffreisetzung, hat sich der Einsatz von Isopropylestern von C₁₂-C₁₈-Carbonsäuren, insbesondere der Einsatz von Isopropylmyristat, und besonders bevorzugt Mischungen von Isopropylmyristat mit C₁₀-C₁₃-Isoparaffin-Mischungen, letztere bevorzugt mit einem Dampfdruck bei 20°C von 10 - 400 Pa, erwiesen.

Erfindungsgemäß bevorzugte Mittel enthalten mindestens einen Ester der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können, in einer Gesamtmenge von 1 - 30 Gew.-%, bevorzugt 5 - 26 Gew.-%, besonders bevorzugt 9 - 24 Gew.-%, außerordentlich bevorzugt 12 - 17 Gew.-%, jeweils bezogen auf das Gewicht der gesamten Zusammensetzung.

Ein weiteres besonders bevorzugtes Esteröl ist Triethylcitrat. Weitere erfindungsgemäß bevorzugte Produkte enthalten Triethylcitrat und mindestens ein C₈-C₁₆-Isoparaffin, ausgewählt aus Isononan, Isodecan, Isoundecan, Isododecan, Isotridecan, Isotetradecan, Isopentadecan und Isohexadecan sowie Mischungen dieser Isoparaffine. Weitere erfindungsgemäß bevorzugte Produkte enthalten Triethylcitrat und mindestens ein C₈-C₁₆-Isoparaffin, ausgewählt aus Isononan, Isodecan, Isoundecan, Isododecan, Isotridecan sowie Mischungen dieser C₈-C₁₆-Isoparaffine. Weitere erfindungsgemäß bevorzugte Produkte enthalten Triethylcitrat und eine Mischung aus Isodecan, Isoundecan, Isododecan und Isotridecan.

Weitere erfindungsgemäß bevorzugte nichtflüchtige Nichtsiliconöle sind ausgewählt aus verzweigten gesättigten oder ungesättigten Fettalkoholen mit 6 - 30 Kohlenstoffatomen. Diese Alkohole werden häufig auch als Guerbet-Alkohole bezeichnet, da sie nach der Guerbet-Reaktion erhältlich sind. Bevorzugte Alkoholöle sind 2-Hexyldecanol, 2-Octyldodecanol und 2-Ethylhexylalkohol. Ebenfalls bevorzugt ist Isostearylalkohol. Weitere bevorzugte nichtflüchtige Öle sind ausgewählt aus Mischungen aus Guerbetalkoholen und Guerbetalkoholestern, z. B. 2-Hexyldecanol und 2-Hexyldecyllaurat.

Der im Folgenden gebrauchte Ausdruck "Triglycerid" meint "Glycerintriester". Weitere erfindungsgemäß bevorzugte nichtflüchtige Öle sind ausgewählt aus den Triglyceriden von linearen oder verzweigten, gesättigten oder ungesättigten, gegebenenfalls hydroxylierten C₈₋₃₀-Fettsäuren, sofern diese unter Normalbedingungen flüssig sind. Besonders geeignet kann die Verwendung natürlicher Öle, z.B. Sojaöl, Baumwollsaatöl, Sonnenblumenöl, Palmöl, Palmkernöl, Leinöl, Mandelöl, Rizinusöl, Maisöl, Rapsöl, Olivenöl, Sesamöl, Distelöl, Weizenkeimöl, Pfirsichkernöl und die flüssigen Anteile des Kokosöls und dergleichen sein. Besonders bevorzugt sind synthetische Triglyceridöle, insbesondere Capric/Caprylic Triglycerides, z. B. die Handelsprodukte Myritol® 318 oder Myritol® 331 (BASF/ BASF) mit unverzweigten Fettsäureresten sowie Glyceryltriisostearin und Glyceryltri(2-ethylhexanoat) mit verzweigten Fettsäureresten. Derartige Triglyceridöle machen bevorzugt einen Anteil von weniger als 50 Gew.-% am Gesamtgewicht aller kosmetischen Öle in der erfindungsgemäßen Zusammensetzung aus. Besonders bevorzugt beträgt das Gesamtgewicht an Triglyceridölen 0,5 - 25 Gew.-%, bevorzugt 1 - 5 Gew.-%, jeweils bezogen auf die gesamte Zusammensetzung.

Weitere erfindungsgemäß besonders bevorzugte nichtflüchtige Nichtsiliconöle sind ausgewählt aus den Dicarbonsäureestern von linearen oder verzweigten C₂-C₁₀-Alkanolen, insbesondere Diisopropyladipat, Di-n-butyladipat, Di-(2-ethylhexyl)adipat, Dioctyladipat, Diethyl-/Di-n-butyl/Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)-succinat.

Weitere erfindungsgemäß besonders bevorzugte nichtflüchtige Nichtsiliconöle sind ausgewählt aus den Anlagerungsprodukten von 1 bis 5 Propylenoxid-Einheiten an ein- oder mehrwertige C₈₋₂₂-Alkanole wie Octanol, Decanol, Decandiol, Laurylalkohol, Myristylalkohol und Stearylalkohol, z. B. PPG-2-Myristylether und PPG-3-Myristylether.

Weitere erfindungsgemäß besonders bevorzugte nichtflüchtige Nichtsiliconöle sind ausgewählt aus den Anlagerungsprodukten von mindestens 6 Ethylenoxid- und/oder Propylenoxid-Einheiten an ein- oder mehrwertige C₃₋₂₂-Alkanole wie Glycerin, Butanol, Butandiol, Myristylalkohol und Stearylalkohol, die gewünschtenfalls verestert sein können, z.B. PPG-14-Butylether, PPG-9-Butylether, PPG-10-Butandiol und PPG-15-Stearylether.

Weitere erfindungsgemäß besonders bevorzugte nichtflüchtige Nichtsiliconöle sind ausgewählt aus den symmetrischen, unsymmetrischen oder cyclischen Estern der Kohlensäure mit C₆-C₂₀-Alkoholen, z. B. Di-n-caprylylcarbonat (Cetiol® CC) oder Di-(2-ethylhexyl)carbonat (Tegosoft DEC). Ester der Kohlensäure mit C₁-C₅-Alkoholen, z. B. Glycerincarbonat oder Propylencarbonat, sind hingegen keine als kosmetisches Öl geeigneten Verbindungen.

Weitere Öle, die erfindungsgemäß bevorzugt sein können, sind ausgewählt aus den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen. Besonders bevorzugt beträgt das Gesamtgewicht an Dimerfettsäureestern 0,5 - 10 Gew.-%, bevorzugt 1 - 5 Gew.-%, jeweils bezogen auf die gesamte Zusammensetzung.

Weitere kosmetische Öle, die erfindungsgemäß besonders bevorzugt sind, sind ausgewählt aus nichtflüchtigen Siliconölen. Erfindungsgemäß bevorzugte nichtflüchtige Siliconöle sind ausgewählt aus linearen Polyalkylsiloxanen mit einer kinematischen Viskosität bei 25°C von mindestens 5 cSt bis 2000 cSt, insbesondere ausgewählt aus linearen Polydimethylsiloxanen mit einer kinematischen Viskosität bei 25°C von 5 cSt bis 2000 cSt, bevorzugt 10 bis 350 cSt, besonders bevorzugt 50 - 100 cSt, wie sie z. B. unter den Handelsnamen Dow Corning® 200 bzw. Xiameter PMX von Dow Corning bzw. Xiameter erhältlich sind. Weitere bevorzugte nichtflüchtige Siliconöle sind Phenyltrimethicone mit einer kinematischen Viskosität bei 25°C von 10 bis 100 cSt, bevorzugt von 15 - 30 cSt sowie Cetyldimethicone.

Erfindungsgemäß bevorzugte natürliche und synthetische Kohlenwasserstoffe sind ausgewählt aus Paraffinölen, Isohexadecan, Isoeicosan, Polyisobutenen und Polydecenen, die z. B. unter der Bezeichnung Emery® 3004, 3006, 3010 oder unter der Bezeichnung Nexbase® 2004G von Nestle erhältlich sind, sowie 1,3-Di-(2-ethylhexyl)-cyclohexan.

Die erfindungsgemäßen und erfindungsgemäß verwendeten Zusammensetzungen enthalten optional weitere Träger-, Hilfs- und Aktivstoffe.

Erfindungsgemäße Antitranspirant-Stifte können in gelierter Form, auf Basis einer W/O-Emulsion, auf Basis einer O/W-Emulsion, auf Basis einer Wasser-Öl-Mehrfach-Emulsion, auf Basis einer Nanoemulsion und auf Basis einer Mikroemulsion vorliegen, wobei die Ölphase mindestens eine Siliconkomponente enthalten oder aus mindestens einer Siliconkomponente bestehen kann. Gelstifte können auf der Basis von Fettsäureseifen, Dibenzylidensorbitol, N-Acylaminosäureamiden, 12-Hydroxystearinsäure, Polyamiden, Polyamidderivaten, Polysacchariden wie Xanthan, Polyglucomannanen, Guar, Konjak, Cellulosen oder Stärken, Polyacrylaten, Polyacrylatderivaten und anderen Gelbildnern formuliert werden.

Roll-on-Applikationen und Cremes können als Wasser-in-ÖI-Emulsion, Öl-in-Wasser-Emulsion, Siliconöl-in-Wasser-Emulsion, Wasser-in-Öl-Mikroemulsion, Öl-in-Wasser-Mikroemulsion, Siliconöl-in-Wasser-Mikroemulsion, alkoholische Lösung, insbesondere ethanolische Lösung, hydroalkoholische Lösung, insbesondere Lösungen mit mehr als 50 Gew.-% eines Wasser-Ethanol-Gemisches, glycolische Lösung, insbesondere als Lösung in 1,2-Propylenglycol, Glycerin, Dipropylenglycol und (unter Normalbedingungen) flüssigen Polyethylenglycolen, hydroglycolische Lösung, Polyol-Lösung, Wasser-Polyol-Lösung und als wässriges Gel vorliegen. Alle genannten Zusammensetzungen können verdickt sein, beispielsweise auf der Basis von Fettsäureseifen, Dibenzylidensorbitol, N-Acylaminosäureamiden, 12-Hydroxystearinsäure, Polyacrylaten vom Carbomer- und Carbopol-Typ, Polyacrylamiden und Polysacchariden, die chemisch und/oder physikalisch modifiziert sein können. Die Mittel können transparent, translucent oder opak sein.

### Lipid- oder Wachsmatrix

Sofern die erfindungsgemäßen Mittel in Form eines Stiftes vorliegen, enthalten sie bevorzugt eine Lipid- oder Wachsmatrix, umfassend mindestens eine Lipid- oder Wachskomponente mit einem Schmelzpunkt > 50°C.

Erfindungsgemäß bevorzugt sind beispielsweise natürliche pflanzliche Wachse, z. B. Candelillawachs, Carnaubawachs, Japanwachs, Zuckerrohrwachs, Ouricourywachs, Korkwachs, Sonnenblumenwachs, Fruchtwachse wie Orangenwachse, Zitronenwachse, Grapefruitwachs, und tierische Wachse, z. B. Bienenwachs, Schellackwachs und Walrat. Im Sinne der Erfindung kann es besonders bevorzugt sein, hydrierte oder gehärtete Wachse einzusetzen. Als Wachskomponente sind auch chemisch modifizierte Wachse, insbesondere die Hartwachse, wie z. B. Montanesterwachse, hydrierte Jojobawachse und Sasolwachse, einsetzbar. Zu den synthetischen Wachsen, die ebenfalls erfindungsgemäß bevorzugt sind, zählen beispielsweise Polyalkylenwachse und Polyethylenglycolwachse, C₂₀-C₄₀-Dialkylester von Dimersäuren, C₃₀₋₅₀-Alkylbienenwachs sowie Alkyl- und Alkylarylester von Dimerfettsäuren.

Eine besonders bevorzugte Wachskomponente ist ausgewählt aus mindestens einem Ester aus einem gesättigten, einwertigen C₁₆-C₆₀-Alkohol und einer gesättigten C₈-C₃₆-Monocarbonsäure. Erfindungsgemäß zählen hierzu auch Lactide, die cyclischen Doppelester von α-Hydroxycarbonsäuren der entsprechenden Kettenlänge. Ester aus Fettsäuren und langkettigen Alkoholen haben sich für die erfindungsgemäße Zusammensetzung als besonders vorteilhaft erwiesen, weil sie der Antitranspirantzubereitung ausgezeichnete sensorische Eigenschaften und dem Stift insgesamt eine hohe Stabilität verleihen. Die Ester setzen sich aus gesättigten verzweigten oder unverzweigten Monocarbonsäuren und gesättigten verzweigten oder unverzweigten einwertigen Alkoholen zusammen. Auch Ester aus aromatischen Carbonsäuren bzw. Hydroxycarbonsäuren (z. B. 12-Hydroxystearinsäure) und gesättigten verzweigten oder unverzweigten Alkoholen sind erfindungsgemäß einsetzbar, sofern die Wachskomponente einen Schmelzpunkt > 50°C hat. Besonders bevorzugt ist, die Wachskomponenten zu wählen aus der Gruppe der Ester aus gesättigten verzweigten oder unverzweigten Alkancarbonsäuren einer Kettenlänge von 12 bis 24 C-Atomen und den gesättigten verzweigten oder unverzweigten Alkoholen einer Kettenlänge von 16 bis 50 C-Atomen, die einen Schmelzpunkt > 50°C haben. Insbesondere können als Wachskomponente C₁₆₋₃₆-Alkylstearate und C₁₈₋₃₈-Alkylhydroxystearoylstearate, C₂₀₋₄₀-Alkylerucate sowie Cetearylbehenat vorteilhaft sein. Das Wachs oder die Wachskomponenten weisen einen Schmelzpunkt >50°C, bevorzugt > 60°C, auf. Eine besonders bevorzugte Ausführungsform der Erfindung enthält als Wachskomponente ein C₂₀-C₄₀-Alkylstearat. Eine weitere besonders bevorzugte Ausführungsform der Erfindung enthält als Wachskomponente Cetearylbehenat, d. h. Mischungen aus Cetylbehenat und Stearylbehenat.

Weitere bevorzugte Lipid- oder Wachskomponenten mit einem Schmelzpunkt > 50°C sind die Triglyceride gesättigter und gegebenenfalls hydroxylierter C₁₂₋₃₀-Fettsäuren, wie gehärtete Triglyceridfette (hydriertes Palmöl, hydriertes Kokosöl, hydriertes Rizinusöl), Glyceryltribehenat (Tribehenin) oder Glyceryltri-12-hydroxystearat, weiterhin synthetische Vollester aus Fettsäuren und Glycolen oder Polyolen mit 2 - 6 Kohlenstoffatomen, solange sie einen Schmelzpunkt oberhalb von 50 °C aufweisen, beispielsweise bevorzugt C₁₈ - C₃₆ Acid Triglyceride (Syncrowax® HGL-C). Erfindungsgemäß ist als Wachskomponente hydriertes Rizinusöl, erhältlich z.B. als Handelsprodukt Cutina® HR, besonders bevorzugt.

Weitere bevorzugte Lipid- oder Wachskomponenten mit einem Schmelzpunkt > 50°C sind die gesättigten linearen C₁₄ - C₃₆-Carbonsäuren, insbesondere Myristinsäure, Palmitinsäure, Stearinsäure und Behensäure sowie Mischungen dieser Verbindungen.

Bevorzugte erfindungsgemäße Antitranspirant-Stifte enthalten eine Lipid- oder Wachskomponente, die ausgewählt ist aus Estern aus einem gesättigten, einwertigen C₁₆-C₆₀-Alkanol und einer gesättigten C₈-C₃₆-Monocarbonsäure, insbesondere Cetylbehenat, Stearylbehenat und C₂₀-C₄₀-Alkylstearat, Glycerintriestern von gesättigten linearen C₁₂ - C₃₀-Carbonsäuren, die hydroxyliert sein können, Candelillawachs, Carnaubawachs, Bienenwachs, gesättigten linearen C₁₄ - C₃₆-Carbonsäuren sowie Mischungen der vorgenannten Substanzen. Besonders bevorzugte Lipid- oder Wachskomponenten-Mischungen sind ausgewählt aus Mischungen von Cetylbehenat, Stearylbehenat, gehärtetem Rizinusöl, Palmitinsäure und Stearinsäure. Weitere besonders bevorzugte Lipid- oder Wachskomponenten-Mischungen sind ausgewählt aus Mischungen von C₂₀-C₄₀-Alkylstearat, gehärtetem Rizinusöl, Palmitinsäure und Stearinsäure. Weitere bevorzugte erfindungsgemäße Antitranspirant-Stifte enthalten Lipid- oder Wachskomponente/n insgesamt in Mengen von 0,5 - 30 Gew.-%, bevorzugt 1 - 25 Gew.-%, bezogen auf die Gesamtzusammensetzung. Besonders bevorzugt enthalten die erfindungsgemäßen Mittel zusätzlich mindestens einen Emulgator und/oder mindestens ein Tensid.

Erfindungsgemäß bevorzugt geeignete Emulgatoren und Tenside sind ausgewählt aus anionischen, kationischen, nichtionischen, amphoteren, insbesondere ampholytischen und zwitterionischen, Emulgatoren und Tensiden.

Tenside sind amphiphile (bifunktionelle) Verbindungen, die aus mindestens einem hydrophoben und mindestens einem hydrophilen Molekülteil bestehen. Der hydrophobe Rest ist bevorzugt eine Kohlenwasserstoffkette mit 8-28 Kohlenstoff-Atomen, die gesättigt oder ungesättigt, linear oder verzweigt sein kann. Besonders bevorzugt ist diese C₈-C₂₈-Alkylkette linear.

Basiseigenschaften der Tenside und Emulgatoren sind die orientierte Absorption an Grenzflächen sowie die Aggregation zu Mizellen und die Ausbildung von lyotrophen Phasen.

Unter anionischen Tensiden werden Tenside mit ausschließlich anionischen Ladungen verstanden; sie enthalten z. B. Carboxylgruppen, Sulfonsäuregruppen oder Sulfatgruppen. Besonders bevorzugte anionische Tenside sind Alkylsulfate, Alkylethersulfate, Acylglutamate und C8-C24-Carbonsäuren sowie deren Salze, die so genannten Seifen.

Unter kationischen Tensiden werden Tenside mit ausschließlich kationischen Ladungen verstanden; sie enthalten z. B. quartäre Ammoniumgruppen. Bevorzugt sind kationische Tenside vom Typ der quartären Ammoniumverbindungen, der Esterquats und der Amidoamine. Bevorzugte quaternäre Ammoniumverbindungen sind Ammoniumhalogenide sowie die unter den INCI-Bezeichnungen Quaternium-27 und Quaternium-83 bekannten Imidazolium-Verbindungen. Weitere erfindungsgemäß verwendbare kationische Tenside stellen die quaternisierten Proteinhydrolysate dar. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Die kationischen Tenside sind in den erfindungsgemäß bevorzugten Mitteln bevorzugt in Anteilen von 0,05 bis 10 Gew.-%, bezogen auf das gesamte Mittel, enthalten.

Die amphoteren Tenside werden in ampholytische Tenside und zwitterionische Tenside unterteilt. Unter ampholytischen Tensiden werden solche oberflächenaktiven Verbindungen verstanden, die sowohl saure (beispielsweise -COOH oder -SO₃H-Gruppen) als auch basische hydrophile Gruppen (beispielsweise Aminogruppen) besitzen und sich also je nach Bedingung sauer oder basisch verhalten. Unter zwitterionischen Tensiden versteht der Fachmann Tenside, die im selben Molekül sowohl eine negative als auch eine positive Ladung tragen.

Beispiele für bevorzugte zwitterionische Tenide sind die Betaine, die N-Alkyl-N,N-dimethylammonium-glycinate, die N-Acyl-aminopropyl-N,N-dimethylammoniumglycinate und die 2-Alkyl-3-carboxymethyl-3-hydroxyethyl-imidazoline mit jeweils 8 bis 24-C-Atomen in der Alkylgruppe.

Beispiele für bevorzugte ampholytische Tenside sind N-Alkylglycine, N-Alkylaminopropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils 8 bis 24 C-Atomen in der Alkylgruppe.

### Öl-in-Wasser-Emulgatoren

Die erfindungsgemäßen Zusammensetzungen, die als Emulsion, insbesondere als Öl-in-Wasser-Emulsion, formuliert sind, enthalten bevorzugt mindestens einen nichtionischen Öl-in-Wasser-Emulgator mit einem HLB-Wert von mehr als 7 bis 20. Hierbei handelt es sich um dem Fachmann allgemein bekannte Emulgatoren, wie sie beispielsweise in Kirk-Othmer, "Encyclopedia of Chemical Technology", 3. Aufl., 1979, Band 8, Seite 913-916, aufgelistet sind. Für ethoxylierte Produkte wird der HLB-Wert nach der Formel HLB = (100 - L): 5 berechnet, wobei L der Gewichtsanteil der lipophilen Gruppen, das heißt der Fettalkyl- oder Fettacylgruppen, in den Ethylenoxidaddukten, ausgedrückt in Gewichtsprozent, ist.

Bei der Auswahl erfindungsgemäß geeigneter nichtionischer Öl-in-Wasser-Emulgatoren ist es besonders bevorzugt, ein Gemisch von nichtionischen Öl-in-Wasser-Emulgatoren einzusetzen, um die Stabilität der erfindungsgemäßen Stiftzusammensetzungen optimal einstellen zu können. Die einzelnen Emulgatorkomponenten liefern dabei einen Anteil zum Gesamt-HLB-Wert oder mittleren HLB-Wert des Öl-in-Wasser-Emulgatorgemisches gemäß ihrem Mengenanteil an der Gesamtmenge der Öl-in-Wasser-Emulgatoren. Erfindungsgemäß beträgt der mittlere HLB-Wert des Öl-in-Wasser- Emulgatorgemisches 10 - 19, bevorzugt 12 - 18 und besonders bevorzugt 14 - 17. Um derartige mittlere HLB-Werte zu erzielen, werden bevorzugt Öl-in-Wasser-Emulgatoren aus den HLB-Wertbereichen 10 - 14, 14 - 16 und gegebenenfalls 16 - 19 miteinander kombiniert. Selbstverständlich können die Öl-in-Wasser-Emulgatorgemische auch nichtionische Emulgatoren mit HLB-Werten im Bereich von > 7 - 10 und 19 - 20 enthalten; derartige Emulgatorgemische können erfindungsgemäß ebenfalls bevorzugt sein. Die erfindungsgemäßen Antitranspirant-Zusammensetzungen können aber in einer anderen bevorzugten Ausführungsform auch nur einen einzigen Öl-in-Wasser-Emulgator mit einem HLB-Wert im Bereich von 10 - 19 enthalten.

Bevorzugte erfindungsgemäße Antitranspirant-Mittel enthalten mindestens einen nichtionischen Öl-in-Wasser-Emulgator, der ausgewählt ist aus ethoxylierten C₈-C₂₄-Alkanolen mit durchschnittlich 10 - 100 Mol Ethylenoxid pro Mol, ethoxylierten C₈-C₂₄-Carbonsäuren mit durchschnittlich 10 - 100 Mol Ethylenoxid pro Mol, Silicon-Copolyolen mit Ethylenoxid-Einheiten oder mit Ethylenoxid- und Propylenoxid-Einheiten, Alkylmono- und -oligoglycosiden mit 8 - 22 Kohlenstoffatomen im Alkylrest und deren ethoxylierten Analoga, ethoxylierten Sterinen, Partialestern von Polyglycerinen mit 2 bis 10 Glycerineinheiten und mit 1 - 4 gesättigten oder ungesättigten, linearen oder verzweigten, gegebenenfalls hydroxylierten C₈ - C₃₀-Fettsäureresten verestert, sofern sie einen HLB-Wert von mehr als 7 aufweisen, sowie Mischungen der vorgenannten Substanzen.

Die ethoxylierten C₈-C₂₄-Alkanole haben die Formel R¹O(CH₂CH₂O)ₙH, wobei R¹ steht für einen linearen oder verzweigten Alkyl- und/oder Alkenylrest mit 8 - 24 Kohlenstoffatomen und n, die mittlere Anzahl der Ethylenoxid-Einheiten pro Molekül, für Zahlen von 10 - 100, bevorzugt 10 - 30 Mol Ethylenoxid an 1 Mol Caprylalkohol, 2-Ethylhexylalkohol, Caprinalkohol, Laurylalkohol, Isotridecylalkohol, Myristylalkohol, Cetylalkohol, Palmitoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen. Auch Addukte von 10 - 100 Mol Ethylenoxid an technische Fettalkohole mit 12 - 18 Kohlenstoffatomen, wie beispielsweise Kokos-, Palm-, Palmkern- oder Talgfettalkohol, sind geeignet.

Die ethoxylierten C₈-C₂₄-Carbonsäuren haben die Formel R¹(OCH₂CH₂)ₙOH, wobei R¹ steht für einen linearen oder verzweigten gesättigten oder ungesättigten Acylrest mit 8 - 24 Kohlenstoffatomen und n, die mittlere Anzahl der Ethylenoxid-Einheiten pro Molekül, für Zahlen von 10 - 100, bevorzugt 10 - 30 Mol Ethylenoxid an 1 Mol Caprylsäure, 2-Ethylhexansäure, Caprinsäure, Laurinsäure, Isotridecansäure, Myristinsäure, Cetylsäure, Palmitoleinsäure, Stearinsäure, Isostearinsäure, Ölsäure, Elaidinsäure, Petroselinsäure, Arachinsäure, Gadoleinsäure, Behensäure, Erucasäure und Brassidinsäure sowie deren technische Mischungen. Auch Addukte von 10 - 100 Mol Ethylenoxid an technische Fettsäuren mit 12 - 18 Kohlenstoffatomen, wie Kokos-, Palm-, Palmkern- oder Talgfettsäure, sind geeignet. Besonders bevorzugt sind PEG-50-monostearat, PEG-100-monostearat, PEG-50-monooleat, PEG-100-monooleat, PEG-50-monolaurat und PEG-100-monolaurat.

Besonders bevorzugt eingesetzt werden die C₁₂-C₁₈-Alkanole oder die C₁₂-C₁₈-Carbonsäuren mit jeweils 10 - 30 Einheiten Ethylenoxid pro Molekül sowie Mischungen dieser Substanzen, insbesondere Ceteth-12, Ceteth-20, Ceteth-30, Steareth-12, Steareth-20, Steareth-30, Laureth-12 und Beheneth-20.

Weiterhin werden bevorzugt C₈ - C₂₂-Alkylmono- und -oligoglycoside eingesetzt. C₈-C₂₂-Alkylmono- und -oligoglycoside stellen bekannte, handelsübliche Tenside und Emulgatoren dar. Ihre Herstellung erfolgt insbesondere durch Umsetzung von Glucose oder Oligosacchariden mit primären Alkoholen mit 8 - 22 Kohlenstoffatomen. Bezüglich des Glycosidrestes gilt, dass sowohl Monoglycoside, bei denen ein cyclischer Zuckerrest glycosidisch an den Fettalkohol gebunden ist, als auch oligomere Glycoside mit einem Oligomerisationsgrad bis etwa 8, bevorzugt 1 - 2, geeignet sind. Der Oligomerisierungsgrad ist dabei ein statistischer Mittelwert, dem eine für solche technischen Produkte übliche Homologenverteilung zugrunde liegt. Produkte, die unter dem Namen Plantacare® erhältlich sind, enthalten eine glucosidisch gebundene C₈-C₁₆-Alkylgruppe an einem Oligoglucosidrest, dessen mittlerer Oligomerisationsgrad bei 1 - 2, insbesondere bei 1,1 - 1,4, liegt. Besonders bevorzugte C₈ - C₂₂-Alkylmono- und -oligoglycoside sind ausgewählt aus Octylglucosid, Decylglucosid, Laurylglucosid, Palmitylglucosid, Isostearylglucosid, Stearylglucosid, Arachidylglucosid und Behenylglucosid sowie Mischungen hiervon. Auch die vom Glucamin abgeleiteten Acylglucamide sind als nicht-ionische Öl-in-Wasser-Emulgatoren geeignet.

Auch ethoxylierte Sterine, insbesondere ethoxylierte Sojasterine, stellen erfindungsgemäß geeignete Öl-in-Wasser-Emulgatoren dar. Der Ethoxylierungsgrad muss größer als 5, bevorzugt mindestens 10 sein, um einen HLB-Wert größer 7 bis 20 aufzuweisen. Geeignete Handelsprodukte sind z. B. PEG-10 Soy Sterol, PEG-16 Soy Sterol und PEG-25 Soy Sterol.

Weiterhin werden bevorzugt Partialester von Polyglycerinen mit 2 bis 10 Glycerineinheiten und mit 1 bis 4 gesättigten oder ungesättigten, linearen oder verzweigten, gegebenenfalls hydroxylierten C₈ - C₃₀-Fettsäureresten verestert, eingesetzt, sofern sie einen HLB-Wert im Bereich von mehr als 7 bis 20 aufweisen. Besonders bevorzugt sind Diglycerinmonocaprylat, Diglycerinmonocaprat, Diglycerinmonolaurat, Triglycerinmonocaprylat, Triglycerinmonocaprat, Triglycerinmonolaurat, Tetraglycerinmonocaprylat, Tetraglycerinmonocaprat, Tetraglycerinmonolaurat, Pentaglycerinmonocaprylat, Pentaglycerinmonocaprat, Pentaglycerinmonolaurat, Hexaglycerinmonocaprylat, Hexaglycerinmonocaprat, Hexaglycerinmonolaurat, Hexaglycerinmonomyristat, Hexaglycerinmonostearat, Decaglycerinmonocaprylat, Decaglycerinmonocaprat, Decaglycerinmonolaurat, Decaglycerinmonomyristat, Decaglycerinmonoisostearat, Decaglycerinmonostearat, Decaglycerinmonooleat, Decaglycerinmonohydroxystearat, Decaglycerindicaprylat, Decaglycerindicaprat, Decaglycerindilaurat, Decaglycerindimyristat, Decaglycerindiisostearat, Decaglycerindistearat, Decaglycerindioleat, Decaglycerindihydroxystearat, Decaglycerintricaprylat, Decaglycerintricaprat, Decaglycerintrilaurat, Decaglycerintrimyristat, Decaglycerintriisostearat, Decaglycerintristearat, Decaglycerintrioleat und Decaglycerintrihydroxystearat.

Besonders bevorzugte erfindungsgemäße Antitranspirantmittel sind dadurch gekennzeichnet, dass der nichtionische Öl-in-Wasser-Emulgator in einer Gesamtmenge von 0,5 - 10 Gew.-%, besonders bevorzugt 1 - 4 Gew.-% und außerordentlich bevorzugt 1,5-3 Gew.-%, bezogen auf die Gesamtzusammensetzung, enthalten ist.

### Wasser-in-Öl-Emulgatoren

Erfindungsgemäß bevorzugte Zusammensetzungen, die als Emulsion oder Stift formuliert sind, enthalten bevorzugt weiterhin mindestens einen nichtionischen Wasser-in-ÖI-Emulgator mit einem HLB-Wert größer 1,0 und kleiner/gleich 7,0, ausgewählt aus den Mono- und Diestern von Ethylenglycol und den Mono-, Di-, Tri- und Tetraestern von Pentaerythrit mit linearen gesättigten Fettsäuren mit 12 - 30, insbesondere 14 - 22 Kohlenstoffatomen, die hydroxyliert sein können, sowie Mischungen hiervon, als Konsistenzgeber und/oder Wasserbinder. Erfindungsgemäß bevorzugt sind die Mono- und Diester. Erfindungsgemäß bevorzugte C₁₂-C₃₀-Fettsäurereste sind ausgewählt aus Laurinsäure-, Myristinsäure-, Palmitinsäure-, Stearinsäure-, Arachinsäure- und Behensäure-Resten; besonders bevorzugt ist der Stearinsäurerest. Erfindungsgemäß besonders bevorzugte nichtionische Wasser-in-ÖI-Emulgatoren mit einem HLB-Wert größer 1,0 und kleiner/ gleich 7,0 sind ausgewählt aus Pentaerythritylmonostearat, Pentaerythrityldistearat, Pentaerythrityltristearat, Pentaerythrityltetrastearat, Ethylenglycolmonostearat, Ethylenglycoldistearat sowie Mischungen hiervon. Erfindungsgemäß besonders bevorzugte Wasser-in-ÖI-Emulgatoren mit einem HLB-Wert größer 1,0 und kleiner/ gleich 7,0 sind zum Beispiel als Handelsprodukte Cutina® PES (INCI: Pentaerythrityl distearate), Cutina® AGS (INCI: Glycol distearate) oder Cutina® EGMS (INCI: Glycol stearate) erhältlich. Diese Handelsprodukte stellen bereits Mischungen aus Mono- und Diestern (bei den Pentaerythritylestern sind auch Tri- und Tetraester enthalten) dar. Erfindungsgemäß kann es bevorzugt sein, nur einen einzigen Wasser-in-ÖI-Emulgator einzusetzen. In einer anderen bevorzugten Ausführungsform enthalten die erfindungsgemäßen Zusammensetzungen Mischungen, insbesondere technische Mischungen, von mindestens zwei Wasser-in-ÖI-Emulgatoren. Unter einer technischen Mischung wird beispielsweise ein Handelsprodukt wie Cutina® PES verstanden. Außer den genannten Wasser-in-ÖI-Emulgatoren auf Basis der Ethylenglycol- oder Pentaerythritylester kann in einer bevorzugten Ausführungsform auch noch mindestens ein weiterer nichtionischer Wasser-in-ÖI-Emulgator mit einem HLB-Wert größer 1,0 und kleiner/gleich 7,0 enthalten sein, dessen Anteil an dem Gesamtgewicht an nichtionischen Wasser-in-ÖI-Emulgatoren mit einem HLB-Wert größer 1,0 und kleiner/gleich 7,0 bevorzugt allerdings nicht größer als 80 % sein sollte. In einer besonders bevorzugten Ausführungsform enthalten die erfindungsgemäßen Zusammensetzungen den mindestens einen zusätzlichen Wasser-in-ÖI-Emulgator mit einem HLB-Wert größer 1,0 und kleiner/ gleich 7,0 nur in einem Gewichtsanteil von maximal 10 % beziehungsweise sind frei von zusätzlichen Wasser-in-ÖI-Emulgatoren. Einige dieser zusätzlichen geeigneten Emulgatoren sind beispielsweise in Kirk-Othmer, "Encyclopedia of Chemical Technology", 3. Aufl., 1979, Band 8, Seite 913, aufgelistet. Für ethoxylierte Addukte lässt sich der HLB-Wert, wie bereits erwähnt, auch berechnen.

Als Wasser-in-ÖI-Emulgator bevorzugt geeignet sind:
- lineare gesättigte Alkanole mit 12 - 30 Kohlenstoffatomen, insbesondere mit 16 - 22 Kohlenstoffatomen, insbesondere Cetylalkohol, Stearylalkohol, Arachidylalkohol, Behenylalkohol und Lanolinalkohol oder Gemische dieser Alkohole, wie sie bei der technischen Hydrierung von pflanzlichen und tierischen Fettsäuren erhältlich sind,
- Ester und insbesondere Partialester aus einem Polyol mit 3 - 6 C-Atomen und linearen gesättigten und ungesättigten Fettsäuren mit 12 - 30, insbesondere 14 - 22 C-Atomen, die hydroxyliert sein können. Solche Ester oder Partialester sind z. B. die Mono- und Diester von Glycerin oder die Monoester von Propylenglycol mit linearen gesättigten und ungesättigten C₁₂ - C₃₀-Carbonsäuren, die hydroxyliert sein können, insbesondere diejenigen mit Palmitin- und Stearinsäure, die Sorbitanmono-, -di- oder -triester von linearen gesättigten und ungesättigten C₁₂ - C₃₀-Carbonsäuren, die hydroxyliert sein können, insbesondere diejenigen von Myristinsäure, Palmitinsäure, Stearinsäure oder von Mischungen dieser Fettsäuren und die Methylglucosemono- und -diester von linearen gesättigten und ungesättigten C₁₂ - C₃₀-Carbonsäuren, die hydroxyliert sein können;
- Sterine, also Steroide, die am C3-Atom des Steroid-Gerüstes eine Hydroxylgruppe tragen und sowohl aus tierischem Gewebe (Zoosterine, z. B. Cholesterin, Lanosterin) wie auch aus Pflanzen (Phytosterine, z. B. Ergosterin, Stigmasterin, Sitosterin) und aus Pilzen und Hefen (Mykosterine) isoliert werden und die niedrig ethoxyliert (1 - 5 EO) sein können;
- Alkanole und Carbonsäuren mit jeweils 8 - 24 C-Atomen, insbesondere mit 16 - 22 C-Atomen, in der Alkylgruppe und 1 - 4 Ethylenoxid-Einheiten pro Molekül, die einen HLB-Wert größer 1,0 und kleiner/gleich 7,0 aufweisen,
- Glycerinmonoether gesättigter und/oder ungesättigter, verzweigter und/oder unverzweigter Alkohole einer Kettenlänge von 8 - 30, insbesondere 12 - 18 Kohlenstoffatomen.
- Partialester von Polyglycerinen mit n = 2 bis 10 Glycerineinheiten und mit 1 bis 5 gesättigten oder ungesättigten, linearen oder verzweigten, gegebenenfalls hydroxylierten C₈ - C₃₀-Fettsäureresten verestert, sofern sie einen HLB-Wert von kleiner/gleich 7 aufweisen,
- sowie Mischungen der vorgenannten Substanzen.

Erfindungsgemäß kann es bevorzugt sein, nur einen einzigen zusätzlichen Wasser-in-ÖI-Emulgator einzusetzen. In einer anderen bevorzugten Ausführungsform enthalten die erfindungsgemäßen Zusammensetzungen Mischungen, insbesondere technische Mischungen, von mindestens zwei zusätzlichen Wasser-in-ÖI-Emulgatoren. Unter einer technischen Mischung wird beispielsweise ein Handelsprodukt wie Cutina® GMS verstanden, das eine Mischung aus Glycerylmonostearat und Glyceryldistearat darstellt.

Besonders vorteilhaft einsetzbare zusätzliche Wasser-in-ÖI-Emulgatoren sind Stearylalkohol, Cetylalkohol, Glycerylmonostearat, insbesondere in Form der Handelsprodukte Cutina® GMS und Cutina® MD (ex BASF), Glyceryldistearat, Glycerylmonocaprinat, Glycerylmonocaprylat, Glycerylmonolaurat, Glycerylmonomyristat, Glycerylmonopalmitat, Glycerylmonohydroxystearat, Glycerylmonooleat, Glycerylmonolanolat, Glyceryldimyristat, Glyceryldipalmitat, Glyceryldioleat, Propylenglycolmonostearat, Propylenglycolmonolaurat, Sorbitanmonocaprylat, Sorbitanmonolaurat, Sorbitanmonomyristat, Sorbitanmonopalmitat, Sorbitanmonostearat, Sorbitansesquistearat, Sorbitandistearat, Sorbitandioleat, Sorbitansesquioleat, Saccharosedistearat, Arachidylalkohol, Behenylalkohol, Polyethylenglycol(2)stearylether (Steareth-2), Steareth-5, Oleth-2, Diglycerinmonostearat, Diglycerinmonoisostearat, Diglycerinmonooleat, Diglycerindihydroxystearat, Diglycerindistearat, Diglycerindioleat, Triglycerindistearat, Tetraglycerinmonostearat, Tetraglycerindistearat, Tetraglycerintristearat, Decaglycerinpentastearat, Decaglycerinpentahydroxystearat, Decaglycerinpentaisostearat, Decaglycerinpentaoleat, Soy Sterol, PEG-1 Soy Sterol, PEG-5 Soy Sterol, PEG-2-monolaurat und PEG-2-monostearat.

Besonders bevorzugte erfindungsgemäße Antitranspirant-Zusammensetzungen sind dadurch gekennzeichnet, dass mindestens ein Wasser-in-ÖI-Emulgator in einer Gesamtmenge von 0,1 - 15 Gew.-%, bevorzugt 0,5 - 8,0 Gew.-%, und besonders bevorzugt 1 - 4 Gew.-%, bezogen auf die Gesamtzusammensetzung, enthalten sind. Weiterhin können auch Mengen von 2 - 3 Gew.-%, bezogen auf das Gesamtgewicht der Zusammensetzung, erfindungsgemäß außerordentlich bevorzugt sein.

Die HLB-Werte können nach Griffin berechnet werden, wie es beispielsweise im RÖMPP Chemie Lexikon, insbesondere in der Online-Version von November 2003, und den dort unter dem Stichwort "HLB-System" zitierten Handbüchern von Fiedler, Kirk-Othmer und Janistyn (H. Janistyn, Handbuch der Kosmetika und Riechstoffe, Hüthig-Verlag Heidelberg, 3. Auflage, 1978, Band 1, Seite 470 und Band 3, Seiten 68 - 78) dargestellt beziehungsweise tabelliert ist. Sofern es in der Literatur unterschiedliche Angaben zum HLB-Wert einer Substanz gibt, sollte derjenige HLB-Wert für die erfindungsgemäße Lehre genutzt werden, der dem nach Griffin berechneten Wert am nächsten kommt. Falls sich auf diese Art und Weise kein eindeutiger HLB-Wert ermitteln lässt, ist der HLB-Wert, den der Hersteller des Emulgators angibt, für die erfindungsgemäße Lehre zu nutzen. Falls auch dies nicht möglich ist, ist der HLB-Wert experimentell zu ermitteln.

Weitere bevorzugte erfindungsgemäße Zusammensetzungen sind dadurch gekennzeichnet, dass der Gesamtgehalt an nichtionischen und ionischen Emulgatoren und/oder Tensiden mit einem HLB-Wert über 8 maximal 20 Gew.-%, bevorzugt maximal 15 Gew.-%, besonders bevorzugt maximal 10 Gew.-%, besonders bevorzugt maximal 7 Gew.-%, weiterhin besonders bevorzugt maximal 4 Gew.-% und außerordentlich bevorzugt maximal 3 Gew.-%, jeweils bezogen auf die gesamte erfindungsgemäße Zusammensetzung, beträgt.

Erfindungsgemäß besonders bevorzugte Zusammensetzungen, die als Wasser-in-ÖI-Emulsion konfektioniert sind, enthalten bevorzugt weiterhin mindestens einen Wasser-in-ÖI-Emulgator. Der mindestens eine Wasser-in-ÖI-Emulgator ist bevorzugt in einer Menge von 0,5 - 10 Gew.-%, besonders bevorzugt 1,0 - 1,5 - 2,5 Gew.-%, jeweils bezogen auf das Gesamtgewicht der Emulsion, enthalten.

Eine erfindungsgemäß besonders bevorzugte Gruppe von Wasser-in-ÖI-Emulgatoren sind die Poly-(C₂-C₃)alkylenglycol-modifizierten Silicone, deren frühere INCI-Bezeichnung Dimethicone Copolyol lautete, mit den aktuellen INCI-Bezeichnungen PEG-x Dimethicone (mit x = 2 - 20, bevorzugt 3 - 17, besonders bevorzugt 11 - 12), Bis-PEG-y Dimethicone (mit y = 3 - 25, bevorzugt 4 - 20), PEG/PPG a/b Dimethicone (wobei a und b unabhängig voneinander für Zahlen von 2 - 30, bevorzugt 3 - 30 und besonders bevorzugt 12 - 20, insbesondere 14 - 18, stehen), Bis-PEG/PPGc/d Dimethicone (wobei c und d unabhängig voneinander für Zahlen von 10 - 25, bevorzugt 14 - 20 und besonders bevorzugt 14 - 16, stehen) und Bis-PEG/PPG-e/f PEG/PPG g/h Dimethicone (wobei e, f, g und h unabhängig voneinander für Zahlen von 10 - 20, bevorzugt 14 - 18 und besonders bevorzugt 16, stehen). Besonders bevorzugt sind PEG/PPG-18/18 Dimethicone, das in einer 1:9- oder 2,5 :7,5 Mischung mit Cyclomethicone oder Dimethicone als DC 3225 C bzw. DC 5225 C , DC 5227-C im Handel erhältlich ist, PEG/PPG-4/12 Dimethicone, das unter der Bezeichnung Abil B 8852 erhältlich ist, sowie Bis-PEG/PPG-14/14 Dimethicone, das in einer Mischung mit Cyclomethicone als Abil EM 97 (Goldschmidt) im Handel erhältlich ist, Bis-PEG/PPG-20/20 Dimethicone, das unter der Bezeichnung Abil B 8832 erhältlich ist, PEG/PPG-5/3 Trisiloxane (Silsoft 305), sowie PEG/PPG-20/23 Dimethicone (Silsoft 430 und Silsoft 440).

Weitere erfindungsgemäß bevorzugte W/O-Emulgatoren sind Poly-(C₂-C₃)alkylenglycol-modifizierte Silicone, die mit C₄-C₁₈-Alkylgruppen hydrophob modifiziert sind, besonders bevorzugt Cetyl PEG/PPG-10/1 Dimethicone (früher: Cetyl Dimethicone Copolyol, erhältlich als Abil EM 90 oder in einer Mischung aus Polyglyceryl-4-isostearat, Cetyl PEG/PPG-10/1 Dimethicone und Hexyllaurat unter der Handelsbezeichnung Abil WE 09), weiterhin Alkyl Methicone Copolyole.

Erfindungsgemäß besonders bevorzugte Zusammensetzungen enthalten weiterhin bevorzugt mindestens einen hautkühlenden Wirkstoff enthalten. Erfindungsgemäß geeignete hautkühlende Wirkstoffe sind beispielsweise Menthol, Isopulegol sowie Mentholderivate, z. B. Menthyllactat, Menthylglycolat, Menthyl Ethyl Oxamate, Menthylpyrrolidoncarbonsäure, Menthylmethylether, Menthoxypropandiol, Menthonglycerinacetal (9-Methyl-6-(1-methylethyl)-1,4-dioxaspiro (4.5)decan-2-methanol), Monomenthylsuccinat und 2-Hydroxymethyl-3,5,5-trimethylcyclohexanol. Als hautkühlende Wirkstoffe bevorzugt sind Menthol, Isopulegol, Menthyllactat, Menthoxypropandiol und Menthylpyrrolidoncarbonsäure sowie Mischungen dieser Substanzen, insbesondere Mischungen von Menthol und Menthyllactat, Menthol, Mentholglycolat und Menthyllactat, Menthol und Menthoxypropandiol oder Menthol und Isopulegol.

Erfindungsgemäß besonders bevorzugt ist, dass mindestens ein hautkühlender Wirkstoff in einer Gesamtmenge von 0,01 - 2 Gew.%, besonders bevorzugt 0,02 - 0,5 Gew.% und außerordentlich bevorzugt 0,05 - 0,2 Gew.%, jeweils bezogen auf das Gesamtgewicht der Zusammensetzung, enthalten ist.

Bevorzugte erfindungsgemäße Zusammensetzungen enthalten weiterhin mindestens ein wasserlösliches mehrwertiges C₂ - C₉-Alkanol mit 2 - 6 Hydroxylgruppen und/oder mindestens ein wasserlösliches Polyethylenglycol mit 3 - 20 Ethylenoxid-Einheiten sowie Mischungen hiervon. Bevorzugt sind diese Komponenten ausgewählt aus 1,2-Propylenglycol, 2-Methyl-1,3-propandiol, Glycerin, 1,2-Butylenglycol, 1,3-Butylenglycol, 1,4-Butylenglycol, Pentylenglycolen wie 1,2-Pentandiol und 1,5-Pentandiol, Hexandiolen wie 1,2-Hexandiol und 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, Erythrit, Sorbit, cis-1,4-Dimethylolcyclohexan, trans-1,4-Dimethylolcyclohexan, beliebige Isomeren-Gemische von cis- und trans-1,4-Dimethylolcyclohexan, sowie Mischungen der vorgenannten Substanzen. Geeignete wasserlösliche Polyethylenglycole sind ausgewählt aus PEG-3, PEG-4, PEG-6, PEG-7, PEG-8, PEG-9, PEG-10, PEG-12, PEG-14, PEG-16, PEG-18 und PEG-20 sowie Mischungen hiervon, wobei PEG-3 bis PEG-8 bevorzugt sind.

Bevorzugte erfindungsgemäße Antitranspirant-Mittel enthalten mindestens ein wasserlösliches mehrwertiges C₂ - C₉-Alkanol mit 2 - 6 Hydroxylgruppen und/ oder mindestens ein wasserlösliches Polyethylenglycol mit 3 - 20 Ethylenoxid-Einheiten, das ausgewählt ist aus 1,2-Propylenglycol, 2-Methyl-1,3-propandiol, Glycerin, 1,2-Butylenglycol, 1,3-Butylenglycol, 1,4-Butylenglycol, Pentylenglycolen wie 1,2-Pentandiol und 1,5-Pentandiol, Hexandiolen wie 1,2-Hexandiol und 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, Erythrit, Sorbit, cis-1,4-Dimethylolcyclohexan, trans-1,4-Dimethylolcyclohexan, beliebige Isomeren-Gemische von cis- und trans-1,4-Dimethylolcyclohexan sowie Mischungen der vorgenannten Substanzen.

Besonders bevorzugte erfindungsgemäße Antitranspirant-Mittel enthalten mindestens ein wasserlösliches mehrwertiges C₂ - C₉-Alkanol mit 2 - 6 Hydroxylgruppen und/ oder mindestens ein wasserlösliches Polyethylenglycol mit 3 - 20 Ethylenoxid-Einheiten in einer Gesamtmenge von 3 - 30 Gew.-%, bevorzugt 8 - 25 Gew.-%, besonders bevorzugt 10 - 18 Gew.-%, enthalten ist, jeweils bezogen auf das Gesamtgewicht des Mittels.

Besonders bevorzugte erfindungsgemäße Antitranspirant-Mittel sind dadurch gekennzeichnet, dass mindestens eine Lipid- oder Wachskomponente mit einem Schmelzpunkt im Bereich von 25 - < 50°C, ausgewählt aus Kokosfettsäureglycerinmono-, -di- und -triestern, Butyrospermum Parkii (Shea Butter) und Estern von gesättigten, einwertigen C₈-C₁₈-Alkoholen mit gesättigten C₁₂-C₁₈-Monocarbonsäuren sowie Mischungen dieser Substanzen, enthalten ist. Diese niedriger schmelzenden Lipid- oder Wachskomponenten ermöglichen eine Konsistenzoptimierung stiftförmiger oder cremeförmiger Produkte und eine Minimierung der sichtbaren Rückstände auf der Haut. Besonders bevorzugt sind Handelsprodukte mit der INCI-Bezeichnung Cocoglycerides, insbesondere die Handelsprodukte Novata® (ex BASF), besonders bevorzugt Novata® AB, ein Gemisch aus C₁₂-C₁₈-Mono-, Di- und Triglyceriden, das im Bereich von 30 - 32°C schmilzt, sowie die Produkte der Softisan-Reihe (Sasol Germany GmbH) mit der INCI-Bezeichnung Hydrogenated Cocoglycerides, insbesondere Softisan 100, 133, 134, 138, 142. Weitere bevorzugte Ester von gesättigten, einwertigen C₁₂-C₁₈-Alkoholen mit gesättigten C₁₂-C₁₈-Monocarbonsäuren sind Stearyllaurat, Cetearylstearat (z. B. Crodamol® CSS), Cetylpalmitat (z. B. Cutina® CP) und Myristylmyristat (z. B. Cetiol® MM).

Weitere besonders bevorzugte erfindungsgemäße Antitranspirant-Mittel sind dadurch gekennzeichnet, dass die mindestens eine Lipid- oder Wachskomponente mit einem Schmelzpunkt im Bereich von 25 - < 50°C in Mengen von 0,01 bis 20 Gew.-%, bevorzugt 3 - 20 Gew.-%, besonders bevorzugt 5 - 18 Gew.-% und außerordentlich bevorzugt 6 - 15 Gew.-%, bezogen auf die Gesamtzusammensetzung, enthalten ist.

Besonders bevorzugte erfindungsgemäße Antitranspirant-Mittel enthalten zur Festigung der Konsistenz und zur Verbesserung der sensorischen Eigenschaften weiterhin mindestens einen festen, wasserunlöslichen teilchenförmigen Füllstoff. In einer außerordentlich bevorzugten Ausführungsform ist dieser Füllstoff ausgewählt aus gegebenenfalls modifizierten Stärken (z. B. aus Mais, Reis, Kartoffeln) und Stärkederivaten, die gewünschtenfalls vorverkleistert sind, insbesondere Stärkederivaten vom Typ DRY FLO®, Cellulose und Cellulosederivaten, Siliciumdioxid, Kieselsäuren, z. B. Aerosil®-Typen, sphärischen Polyalkylsesquisiloxan-Partikeln (insbesondere Aerosil® R972 und Aerosil® 200V von Degussa), Kieselgelen, Talkum, Kaolin, Tonen, z. B. Bentoniten, Magnesiumaluminiumsilikaten, Bornitrid, Lactoglobulinderivaten, z. B. Natrium-C₈₋₁₆-Isoalkyl-succinyllactoglobulinsulfonat, von Brooks Industries erhältlich als Handelsprodukt Biopol® OE, Glaspulvern, Polymerpulvern, insbesondere aus Polyolefinen, Polycarbonaten, Polyurethanen, Polyamiden, z. B. Nylon, Polyestern, Polystyrolen, Polyacrylaten, (Meth)acrylat- oder (Meth)acrylat-Vinyliden-Copolymeren, die vernetzt sein können, oder Siliconen, sowie Mischungen dieser Substanzen. Polymerpulver auf Basis eines Polymethacrylat-Copolymers sind z. B. als Handelsprodukt Polytrap® 6603 (Dow Corning) erhältlich. Andere Polymerpulver, z. B. auf Basis von Polyamiden, sind unter der Bezeichnung Orgasol® 1002 (Polyamid-6) und Orgasol® 2002 (Polyamid-12) von Elf Atochem erhältlich. Weitere Polymerpulver, die sich für den erfindungsgemäßen Zweck eignen, sind z. B. Polymethacrylate (Micropearl® M von SEPPIC oder Plastic Powder A von NIKKOL), Styrol-Divinylbenzol-Copolymeren (Plastic Powder FP von NIKKOL), Polyethylen- und Polypropylen-Pulver (ACCUREL® EP 400 von AKZO) oder auch Siliconpolymere (Silicone Powder X2-1605 von Dow Corning).

Besonders bevorzugte erfindungsgemäße Antitranspirant-Mittel enthalten mindestens einen festen, wasserunlöslichen teilchenförmigen Füllstoff in einer Gesamtmenge von 0,01 bis 30 Gew.-%, bevorzugt 5 - 20 Gew.-%, besonders bevorzugt 8 - 15 Gew.-%, jeweils bezogen auf die Gesamtzusammensetzung.

Besonders bevorzugte erfindungsgemäße Antitranspirant-Mittel enthalten weiterhin mindestens einen Riechstoff. Die Definition eines Riechstoffs im Sinne der vorliegenden Anmeldung stimmt überein mit der fachmännisch üblichen Definition, wie sie dem RÖMPP Chemie Lexikon, Stand Dezember 2007, entnommen werden kann. Danach ist ein Riechstoff eine chemische Verbindung mit Geruch und/oder Geschmack, der die Rezeptoren der Haarzellen des olfaktorischen Systems erregt (adäquater Reiz). Die hierzu notwendigen physikalischen und chemischen Eigenschaften sind eine niedrige Molmasse von maximal 300 g/mol, ein hoher Dampfdruck, minimale Wasser- und hohe Lipidlöslichkeit sowie schwache Polarität und das Vorliegen mindestens einer osmophoren Gruppe im Molekül. Um flüchtige, niedermolekulare Substanzen, die üblicherweise und auch im Sinne der vorliegenden Anmeldung nicht als Riechstoff, sondern vornehmlich als Lösemittel angesehen und verwendet werden, wie beispielsweise Ethanol, Propanol, Isopropanol und Aceton, von erfindungsgemäßen Riechstoffen abzugrenzen, weisen erfindungsgemäße Riechstoffe eine Molmasse von 74 bis 300 g/mol auf, enthalten mindestens eine osmophore Gruppe im Molekül und weisen einen Geruch und/oder Geschmack auf, das heißt, sie erregen die Rezeptoren der Haarzellen des olfaktorischen Systems.

Als Riechstoffe können Parfüme, Parfümöle oder Parfümölbestandteile eingesetzt werden. Parfümöle bzw. Duftstoffe können erfindungsgemäß einzelne Riechstoffverbindungen, z. B. die synthetischen Produkte vom Typ der Ester, Ether, Aldehyde, Ketone, Alkohole und Kohlenwasserstoffe sein. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat, Melusat und Jasmecyclat. Zu den Ethern zählen beispielsweise Benzylethylether und Ambroxan , zu den Aldehyden z.B. die linearen Alkanale mit 8 - 18 C-Atomen, Citral, Citronellal, Citronellyloxy-acetaldehyd, Cyclamenaldehyd, Lilial und Bourgeonal, zu den Ketonen z.B. die Jonone, alpha-Isomethylionon und Methylcedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene wie Limonen und Pinen. Bevorzugt werden jedoch Mischungen verschiedener Riechstoffe verwendet, die gemeinsam eine ansprechende Duftnote erzeugen.

Besonders bevorzugte erfindungsgemäße Antitranspirant-Mittel enthalten mindestens eine Riechstoffkomponente in einer Gesamtmenge von 0,00001 bis 10 Gew.-%, bevorzugt 0,5 - 7 Gew.-%, außerordentlich bevorzugt 1 - 6 Gew.-%, jeweils bezogen auf das Gesamtgewicht des Mittels.

Ein weiterer Gegenstand der vorliegenden Anmeldung ist ein Verfahren zur nicht-therapeutischen, kosmetischen schweißhemmenden Behandlung des Körpers, bei dem ein schweißhemmendes kosmetisches Mittel, das in einem kosmetisch verträglichen Träger mindestens ein schweißhemmendes Aluminiumsalz in einer Gesamtmenge von 2 - 40 Gew.-%, bevorzugt 8 - 35 Gew.-%, besonders bevorzugt 10 - 28 Gew.-% und außerordentlich bevorzugt 12 - 20 Gew.-%, wobei sich die Gew.-%-Angaben auf das Gesamtgewicht der kristallwasserfreien und ligandenfreien Aktivsubstanz (USP) in der Zusammensetzung beziehen, und zusätzlich dazu Methansulfonsäure und/oder mindestens einem physiologisch verträglichen Salz der Methansulfonsäure; bevorzugt in einer Gesamtmenge von 0,05 - 5 Gew.-%, bevorzugt 0,1 - 3 Gew.-%, besonders bevorzugt 0,5 - 2,5 Gew.-%, außerordentlich bevorzugt 1 - 2 Gew.-%, enthält, auf die Haut, insbesondere die Haut der Achselhöhlen, aufgetragen wird, wobei sich die Gew.-%-Angaben jeweils auf das Gesamtgewicht des Mittels beziehen.

Bezüglich bevorzugter Ausführungsformen des erfindungsgemäßen Verfahrens gilt mutatis mutandis das zu den erfindungsgemäßen Mitteln Gesagte.

Ein weiterer Gegenstand der vorliegenden Anmeldung ist ein Verfahren zur Verhinderung und/oder Reduzierung von durch Antitranspirantien und Antitranspirantwirkstoffe verursachten Textilverfärbungen und/oder Textilflecken, wobei das Verfahren die folgenden Verfahrensschritte umfasst:
(a) Herstellung eines schweißhemmenden kosmetischen Mittels durch Vermischen mindestens eines schweißhemmenden Aluminiumsalzes, das bevorzugt Zirconium-frei ist, in einer Gesamtmenge von 2 - 40 Gew.-%, bevorzugt 8 - 35 Gew.-%, besonders bevorzugt 10 - 28 Gew.-% und außerordentlich bevorzugt 12 - 20 Gew.-%, wobei sich die Gew.-%-Angaben auf das Gesamtgewicht der kristallwasserfreien und ligandenfreien Aktivsubstanz (USP) in dem Mittel beziehen, mit einem kosmetisch verträglichen Träger und mit Methansulfonsäure der folgenden Formel (MS-1) 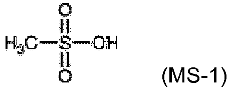 oder mindestens einem physiologisch verträglichen Salz der Methansulfonsäure, wobei die Methansulfonsäure oder das/die Salz/e hiervon bevorzugt in einer Gesamtmenge von 0,05 - 5 Gew.-%, bevorzugt 0,1 - 3 Gew.-%, besonders bevorzugt 0,5 - 2,5 Gew.-%, außerordentlich bevorzugt 1 - 2 Gew.-%, enthalten ist/sind, wobei sich die Gew.-%-Angaben jeweils auf das Gesamtgewicht des Mittels beziehen,
(b) Auftragen des schweißhemmenden kosmetischen Mittels auf die Haut, insbesondere die Haut der Achselhöhlen,
(c) Tragen eines Textilkleidungsstücks über der behandelten Haut, und
(d) Waschen des Textilkleidungsstücks, insbesondere mehrmaliges Waschen des Textilkleidungsstücks, wobei nach dem Waschen, insbesondere dem mehrmaligen Waschen, keine oder verminderte Textilverfärbungen und/oder Textilflecken auftreten.

Bezüglich bevorzugter Ausführungsformen des erfindungsgemäßen Verfahrens zur Verhinderung und/oder Reduzierung von Textilverfärbungen und/oder Textilflecken gilt mutatis mutandis das zu den erfindungsgemäßen Mitteln Gesagte.

### Experimenteller Teil

### Wasserfreie Antitranspirant-Zusammensetzungen

Als erfindungsgemäßes Testprodukt wurde eine ölige Suspension, bestehend aus 14,3 Gew.-% aktiviertem Aluminiumchlorhydrat, 2 Gew.-% der zu prüfenden erfindungsgemäß verwendeten Methansulfonsäure der Formel (MS-1) sowie 65,9 Gew.-% 2-Ethylhexylpalmitat, 5,4 % Triethylcitrat, 3,9% Bentone 38 V CG, 7,2 Gew.-% Parfümöl und 1,3% Propylenecarbonat hergestellt (E-1). Eine derartige Suspension ist unter anderem repräsentativ für wasserfreie Antitranspirant-Rollons und wasserfreie Antitranspirant-Wachsstifte.

Als nicht erfindungsgemäßes Vergleichsprodukt wurde eine Suspension, bestehend aus 14,3 Gew.-% aktiviertem Aluminiumchlorhydrat und 67,9 Gew.-% 2-Ethylhexylpalmitat, 5,4 % Triethylcitrat, 3,9% Bentone 38 V CG, 1,3% Propylenecarbonat und 7,2 Gew.-% Parfümöl hergestellt (V-1).

**Tabelle 1: Verwendete Test- und Vergleichsprodukte (Mengenangaben in Gew.-%)**

| | V-1 | E-1 |
|---|---|---|
| aktiviertes Aluminiumchlorhydrat (AACH) | 14,3 | 14,3 |
| 2-Ethylhexylpamitat | 67,9 | 65,9 |
| Triethylcitrat | 5,4 | 5,4 |
| Bentone 38 V CG | 3,9 | 3,9 |
| Propylencarbonat | 1,3 | 1,3 |
| Parfüm | 7,2 | 7,2 |
| Methansulfonsäure | -- | 2 |

### Versuchsdurchführung

0,3 Gramm des jeweiligen Test- oder Vergleichsprodukts wurde direkt auf ein 10 x10 cm² großes Stück hellblauen Baumwollstoff (Polo Jersey, gewebt) aufgebracht, das auf ein Waffelpiquehandtuch geheftet war. Nach einer Stunde Wartezeit wurde 1 ml einer künstlichen Schweißmischung (MgCl₂, CaCl₂, KCl, NaCl, Na₂SO₄, NaH₂PO₄, Glycin, Glucose, Milchsäure, Harnstoff; pH 5,2) aufgetragen und das Textil nach 24 Stunden Wartezeit (Alterung) in einem standardardisierten, haushaltsüblichen Waschprozess gewaschen (Miele W 1714) und maschinell getrocknet (Miele T 7644C Tabelle 2: Weitere Bedingungen der Waschversuche

| | | |
|---|---|---|
| Beladung Waschmaschine: | 3,5 kg | |
| Wassermenge: | 17 Liter | |
| Temperatur: | 40°C | |
| Zeit Hauptwaschgang: | 1h | |
| Vorwäsche: | ohne | |
| Nachspülen: | 4x | |
| Waschmittel: | Spee Color Gel | |
| | Charge: HH06.1.1UWM1.08:58 | |
| Einwaage Waschmittel: | 75ml (70 gr) | |
| Weichspüler: | ohne | |
| Trocknerprogramm: | Extra Trocken - Baumwolle | |

Die Produktauftragung und Waschung wurde insgesamt achtmal mit dem gleichen Textil wiederholt (8 Anschmutz/Waschzyklen). Die Bewertung der Textilanschmutzung erfolgte visuell durch geschulte Laboranten anhand von Referenzbeispielen. Die Skala reichte von 0 (keine Flecken) bis 4 (sehr starke Fleckenbildung). Die Bewertung erfolgte direkt nach Abschluss der Waschreihe.

**Tabelle 3: Ergebnisse der visuellen Rückstandsbewertung**

| **Produkt** | **nach 8. Waschen** | | |
|---|---|---|---|
| | **weiß** | **fettig** | **gelb** |
| V-1 | 0 | 4 | 0 |
| E-1 | 0 | 2,5 | 0 |

Das erfindungsgemäße Testprodukt E-1 mit 2 Gew.-% Methansulfonsäure zeigte gegenüber der Vergleichsrezeptur V-1 ohne Methansulfonsäure (Tabelle 1) eine deutlich reduzierte Bildung fettiger Flecken auf hellblauem Textil (Tabelle 3).

### Wasserhaltige Antitranspirant-Zusammensetzungen

Als erfindungsgemäßes Testprodukt wurde eine wässrige Lösung, bestehend aus 20 Gew.-% Aluminiumchlorhydrat, 2 Gew.-% Methansulfonsäure sowie 78 Gew.-% Wasser, hergestellt (E-2). Eine derartige Lösung ist unter anderem repräsentativ für wasserhaltige Antitranspirant-Emulsionen (Antitranspirant-Rollons; Antitranspirant-Stifte).

Als nicht erfindungsgemäßes Vergleichsprodukt wurde eine Lösung, bestehend aus 20 Gew.-% Aluminiumchlorhydrat und 80 Gew.-% Wasser, verwendet (V).

**Tabelle 4: Verwendete Test- und Vergleichsprodukte (Mengenangaben in Gew.-%)**

| | V-2 | E-2 |
|---|---|---|
| Aluminiumchlorhydrat (ACH) | 20 | 20 |
| Wasser | 80 | 78 |
| Methansulfonsäure | -- | 2 |

Die Waschversuche wurden analog zur Testreihe mit den wasserfreien Zusammensetzungen durchgeführt.

**Tabelle 5: Ergebnisse der visuellen Rückstandsbewertung**

| **Produkt** | **nach 8. Waschen** | | |
|---|---|---|---|
| | **weiß** | **fettig** | **gelb** |
| V-2 | 4 | 0 | 0 |
| E-2 | 2 | 0 | 0 |

Das erfindungsgemäße Testprodukt E-2 mit 2 Gew.-% Methansulfonsäure zeigte gegenüber der Vergleichsrezeptur V-2 ohne Methansulfonsäure (Tabelle 4) eine deutlich reduzierte Bildung weißer Flecken auf hellblauem Textil (Tabelle 5).

### Wasserhaltige Antitranspirant-Öl-in-Wasser-Emulsion

Als erfindungsgemäßes Testprodukt wurde eine Öl-in-Wasser-Emulsion folgender Zusammensetzung E-3 hergestellt:

**Tabelle 6: Erfindungsgemäße und nicht erfindungsgemäße Testemulsion (O/W); Mengen in Gew.-%**

| | V-3 | E-3 |
|---|---|---|
| ALUMINUM CHLOROHYDRATE | 20,0 | 20,0 |
| STEARETH-2 | 2,4 | 2,4 |
| STEARETH-21 | 1,6 | 1,6 |
| PARFUM | 1,2 | 1,2 |
| PPG-15 STEARYL ETHER | 0,5 | 0,5 |
| ALUMINUM STARCH OCTENYLSUCCINATE | 0,1 | 0,1 |
| Isopropyl myristat | 0,1 | 0,1 |
| Vitamin E Acetat | 0,05 | 0,05 |
| EDTA Pulver | 0,095 | 0,095 |
| Methansulfonsäure | -- | 0,5 |
| Wasser | ad 100,0 | ad 100,0 |

Eine derartige Lösung ist für wasserhaltige Antitranspirant-Emulsionen (Antitranspirant-Rollons; Antitranspirant-Stifte) repräsentativ.

Die Waschversuche wurden ähnlich zu den oben dargestellten Testreihen durchgeführt; allerdings wurden mehr Waschzyklen durchgeführt und die Textilien wurden durch 14 Tage Lagerung gealtert. Hierdurch lässt sich die Entwicklung gelber Flecken nachstellen und evaluieren.

### Versuchsdurchführung

0,3 Gramm des jeweiligen Test- oder Vergleichsprodukts wurde direkt auf ein 9,5 x9,5 cm² großes Stück weißen Baumwollstoff (T-Shirt-Stoff, gestrickt) aufgebracht, das auf ein Waffelpiquehandtuch geheftet war. Nach einer Stunde Wartezeit wurde 1 ml einer künstlichen Schweißmischung (MgCl₂, CaCl₂, KCl, NaCl, Na₂SO₄, NaH₂PO₄, Glycin, Glucose, Milchsäure, Harnstoff; pH 5,2) aufgetragen und das Textil nach 24 Stunden Wartezeit (Alterung) gewaschen (Miele W 1714) und maschinell getrocknet (Miele T 7644C). Insgesamt wurde dieser Anschmutz-/Waschzyklus vierzehnmal wiederholt.

**Tabelle 7: Weitere Bedingungen der Waschversuche**

| | | |
|---|---|---|
| Beladung Waschmaschine: | 3,5 kg | |
| Wassermenge: | 17 Liter | |
| Temperatur: | 40°C | |
| Zeit Hauptwaschgang: | 1h | |
| Vorwäsche: | ohne | |
| Nachspülen: | 4x | |
| Waschmittel: | Persil Universalpulver Gold mit Leuchtkraftformel | |
| | Wero 2020110023 | |
| Einwaage Waschmittel: | 135ml (80 gr) | |
| Weichspüler: | ohne | |
| Trocknerprogramm: | Extra Trocken - Baumwolle | |

Die Produktauftragung und Waschung wurde insgesamt vierzehnmal mit dem gleichen Textil wiederholt. Die Bewertung der Textilanschmutzung erfolgte visuell durch geschulte Laboranten anhand von Referenzbeispielen. Die Skala reichte von 0 (keine Flecken) bis 4 (sehr starke Fleckenbildung). Die Bewertung erfolgte 14 Tage nach Abschluss der Waschreihe.

**Tabelle 8: Ergebnisse der visuellen Rückstandsbewertung**

| **Produkt** | **nach 14 Wäschen + 14 Tage Alterung** | | |
|---|---|---|---|
| | **weiß** | **fettig** | **gelb** |
| V-3 | 0 | 0 | 2 |
| E-3 | 0 | 0 | 1 |

Das erfindungsgemäße Testprodukt E-3 mit 0,5 Gew.-% Methansulfonsäure zeigte gegenüber der Vergleichsrezeptur V-3 ohne Methansulfonsäure (Tabelle 6) eine deutlich reduzierte Bildung gelber Flecken auf weißem Textil (Tabelle 8).

In einem weiteren Waschversuch mit einer wasserfreien Suspension auf Basis einer Cyclopentasiloxan/Isopropylmyristat-Ölmischung und 33 Gew.-% aktiviertem Aluminiumchlorhydrat (beispielhaft für eine wasserfreie Roll on Suspension) wurde eine deutliche Reduktion von weißen Flecken auf hellblauem Baumwollstoff durch einen Gehalt an 2 Gew.-% Methansulfonsäure gezeigt Ausführungsbeispiele

Die nachfolgenden Formulierungsbeispiele sollen den Gegenstand der Erfindung erläutern, ohne ihn hierauf zu beschränken.

### 1. Erfindungsgemäße schweißhemmende Suspensionsstifte (Mengenangaben in Gew.-%)

| | 1.1 | 1.2 | 1.3 |
|---|---|---|---|
| Stearyl Alcohol | 24,0 | 24,0 | 24,0 |
| Anuminium Zirconium Pentachlorohydrex GLY | 22,0 | 22,0 | 22,0 |
| PPG-14 Butyl Ether | 10,0 | 10,0 | 10,0 |
| gehärtetes Rizinusöl (z. B. Cutina HR) | 3,0 | 3,0 | 3,0 |
| Myristylmyristat | 1,5 | 1,5 | 1,5 |
| DL-Menthol | 0,2 | 0,2 | 0,2 |
| Eucalyptol | 0,2 | 0,2 | 0,2 |
| Anethol | 0,2 | 0,2 | 0,2 |
| Silica Dimethyl Silylate | 1,4 | 1,4 | 1,4 |
| Silica | 0,3 | 0,3 | 0,3 |
| Methansulfonsäure | 2,0 | 2,5 | 1,0 |
| Parfum | 2,0 | 2,0 | 2,0 |
| Cyclopentasiloxan | ad 100 | Ad 100 | ad 100 |

| | 1.4 | 1.5 | 1.6 |
|---|---|---|---|
| Hydrogenated Castor Oil | 1,5 | 1,5 | 1,5 |
| Stearylalkohol | 18 | 18 | 18 |
| Novata AB | 4 | 4 | 4 |
| PPG-14 Butylether | 15,3 | 15,3 | 15,3 |
| Al-Zr-tetrachlorohydrex Gly (USP; zahlenmittl. Partikelgröße 3 - 20 µm) | 17,6 | 17,6 | 17,6 |
| Kristallwasser und (sofern vorhanden) Glycin | 4,4 | 4,4 | 4,4 |
| Talkum | 3 | 3 | 3 |
| Parfum | 1 | 1 | 1 |
| Eumulgin B1 | 3 | 3 | 3 |
| Methansulfonsäure | 2,0 | 0,1 | 3,0 |
| Cyclomethicone (mind. 95 Gew.-% Cyclopentasiloxan) | ad 100 | ad 100 | ad 100 |

### 2. Erfindungsgemäße Antitranspirant-Stifte in Form einer Öl-in-Wasser-Emulsion (Mengenangaben in Gew.-%)

| Beispiel Nr. | 2.1 | 2.2 |
|---|---|---|
| Cutina ® AGS | 2,5 | 2,5 |
| Cutina® FS45 | 3,5 | 3,5 |
| Eumulgin® B2 | 0,8 | 0,8 |
| Eumulgin® B3 | 0,8 | 0,8 |
| Diisopropyladipat | 6 | 6 |
| Novata® AB | 4 | 4 |
| Cutina® CP | 5 | 5 |
| Cutina® HR | 4 | 4 |
| Kesterwachs K62 | 5 | 5 |
| Locron® L (ACH-Lösung 50%ig) | 40 | 40 |
| Talkum Pharma G | 10 | 10 |
| Parfüm | 1,2 | 1,2 |
| 2-Benzylheptan-1-ol | - | 0,3 |
| Sensiva SC 50 | 0,6 | 0,6 |
| Methansulfonsäure | 2,0 | 3,0 |
| 1,2-Propandiol | 10 | 10 |
| Wasser, vollentsalzt | ad 100,0 | ad 100,0 |

### Beispiel 3: Erfindungsgemäße Antitranspirant-Emulsion (O/W), Angaben in Gew.-%)

| INGREDIENTS | Gew.-%-Anteil |
|---|---|
| ALUMINUM CHLOROHYDRATE | 20,0 |
| STEARETH-2 | 2,4 |
| STEARETH-21 | 1,6 |
| PARFUM | 1,2 |
| PPG-15 STEARYL ETHER | 0,5 |
| ALUMINUM STARCH OCTENYLSUCCINATE | 0,1 |
| Methansulfonsäure | 2,0 |
| Novata AB | 0,5 |
| Wasser | ad 100,0 |

Die Emulsion gemäß Beispiel 3 wurde in einen Roll-on-Applikator abgefüllt.

### 4. Translucente Antitranspirant-Mikroemulsionen (Angaben in Gew.-%)

| | 4.1 | 4.2 | 4.3 |
|---|---|---|---|
| Plantaren® 1200 | 1,71 | 1,71 | - |
| Plantaren® 2000 | 1,14 | 1,39 | 2,40 |
| Glycerinmonooleat | 0,71 | 0,71 | - |
| Dioctylether | 4,00 | 4,00 | 0,09 |
| Octyldodecanol | 1,00 | 1,00 | 0,02 |
| Parfümöl | 1,00 | 1,00 | 1,00 |
| Aluminiumchlorohydrat | 8,00 | 5,00 | 5,00 |
| 1,2-Propylenglycol | 5,00 | 5,00 | - |
| Glycerin | - | - | 5,00 |
| 2-Benzylheptan-1-ol | 0,5 | - | - |
| Triethylcitrat | - | 0,5 | 0,5 |
| Triclosan | 0,1 | - | - |
| Methansulfonsäure | 1,0 | 2,0 | 2,5 |
| Wasser | ad 100 | ad 100 | ad 100 |

### 5. Antitranspirant Roll-ons (Angaben in Gew.-%)

| | 5.1 | 5.2 |
|---|---|---|
| Ethanol 96 %-ig,(DEP vergällt) | 30,0 | 30,0 |
| Mergital® CS 11 | 2,0 | 2,0 |
| Eumulgin® B 3 | 2,0 | 2,0 |
| Aluminiumchlorohydrat | 20,0 | 20,0 |
| Hydroxyethylcellulose | 0,5 | 0,5 |
| Methansulfonsäure | 2,5 | 0,5 |
| Cocamidopropyl PG-Dimonium Chloride Phosphate | 0,2 | - |
| Parfümöl | 0,8 | 0,8 |
| Wasser | ad 100 | ad 100 |

### 6. Antitranspirant-Tücher (Beispiele Nr. 6.1. - 6.2)

Für die erfindungsgemäße Ausführungsform als Antitranspirant-Tuch wurde ein einlagiges Substrat aus 100 % Viskose mit einem Flächengewicht von 50 g/m² mit jeweils 75 g der Beispielemulsionen 5.1 bzw. 5.2 pro Quadratmeter oder mit jeweils 75 g der Beispielzusammensetzungen 4.1 bzw. 4.2 beaufschlagt, in Tücher geeigneter Größe geschnitten und in Sachets verpackt.

### 7. Antitranspirant-Roll-on

| | 7.1 | 7.2 |
|---|---|---|
| Aluminiumchlorohydrat 50% in Wasser (Locron L) | 16,0 | 16,0 |
| Ethanol 96% | 28,0 | 28,0 |
| Hydroxyethylcellulose | 0,3 | 0,3 |
| Eumulgin B1 | 2,0 | 2,0 |
| Eumulgin B3 | 2,0 | 2,0 |
| Methansulfonsäure | 2,5 | 1,5 |
| EDTA | - | 0,05 |
| Parfum | 1,0 | 1,0 |
| Wasser | ad 100 | ad 100 |

### 8. Antitranspirant-Stift in Form einer Wasser-in-ÖI-Emulsion

| | 8.1 | 8.2 |
|---|---|---|
| Aluminiumchlorohydrat 50% in Wasser | 35,6 | 35,6 |
| 1,2-Propylenglycol | 13,0 | 13,0 |
| Cyclohexasiloxan | 6,0 | 6,0 |
| Finsolv TN | 8,0 | 8,0 |
| Abil EM 90 | 1,2 | 1,2 |
| Polyethylen-Wachs mit einem Molgewicht von 500 g/mol und einem Schmelzpunkt im Bereich von 83 - 91 °C | 10,0 | 10,0 |
| Polyalphaolefin-Wachs mit einem Molgewicht von 1800 g/mol und einem Schmelzpunkt von 41 °C | 0,1 | 0,1 |
| Methansulfonsäure | 2,0 | 0,5 |
| EDTA | - | 0,05 |
| Wasser | 25,0 | 25,0 |
| Parfum | 1,0 | 1,0 |

### 9. Klares AT-Gel

| | 9.1 | 9.2 |
|---|---|---|
| Cyclopentasiloxan | 14,0 | 14,0 |
| Abil EM 97 | 3,0 | 3,0 |
| Ethanol 96% | 10,0 | 10,0 |
| Aluminiumchlorohydrat 50% in Wasser (Locron L) | 40,0 | 40,0 |
| 1,2-Propylenglycol | 20,3 | 20,3 |
| Wasser | 11,6 | 11,6 |
| Methansulfonsäure | 2,0 | 0,5 |
| EDTA | - | 0,075 |
| Parfum | 1,0 | 1,0 |

### Liste der verwendeten Rohstoffe

| Komponente | INCI | Lieferant/Hersteller |
|---|---|---|
| Abil EM 90 | CETYL PEG/PPG-10/1 Dimethicone | Evonik |
| Abil EM 97 | Bis-PEG/PPG-14/14 Dimethicone, Cyclomethicone | Evonik |
| Cutina® CP | Cetyl Palmitate | BASF |
| Cutina® FS45 | Palmitic Acid, Stearic Acid | BASF |
| Cutina® HR | Hydrogenated Castor Oil | BASF |
| Dow Corning ® 245 | Cyclopentasiloxan | Dow Corning |
| Dow Corning ES-5227 DM | Dimethicone, PEG/PPG-18/18 Dimethicone im Gewichtsverhältnis 3:1 | Dow Corning |
| Eumulgin® B1 | Ceteareth-12 | BASF |
| Eumulgin® B2 | Ceteareth-20 | BASF |
| Eumulgin® B3 | Ceteareth-30 | BASF |
| Kesterwachs K62 | Cetearyl Behenate | Koster Keunen |
| Finsolv TN | C12-15 Alkyl Benzoate | Innospec |
| Locron L (ACH-Lösung 50%ig) | Aluminum Chlorohydrate | Clariant |
| Mergital® CS 11 | Ceteareth-11 | BASF |
| Novata® AB | Cocoglycerides (Schmelzpunkt 30 - 32 °C) | BASF |
| Plantaren® 1200 | LAURYL GLUCOSIDE, ca. 50 % AS | BASF |
| Plantaren® 2000 | DECYL GLUCOSIDE, ca. 50 % AS | BASF |
| Sensiva® SC 50 | 2-Ethylhexylglycerinether | Schülke & Mayr |

## Patentansprüche

1. Schweißhemmendes kosmetisches Mittel zur Nonaerosol-Anwendung, enthaltend in einem kosmetisch verträglichen Träger
a) mindestens ein schweißhemmendes Aluminiumsalz in einer Gesamtmenge von 2 - 40 Gew.-%, bevorzugt 8 - 35 Gew.-%, besonders bevorzugt 10 - 28 Gew.-% und außerordentlich bevorzugt 12 - 20 Gew.-%, wobei sich die Gew.-%-Angaben auf das Gesamtgewicht der kristallwasserfreien und ligandenfreien Aktivsubstanz (USP) in dem Mittel beziehen,
und zusätzlich dazu
b) Methansulfonsäure der folgenden Formel (MS-1) 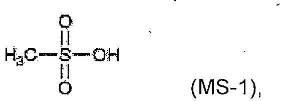
wobei das schweißhemmende Aluminiumsalz ausgewählt ist aus den wasserlöslichen adstringierenden anorganischen Salzen von Aluminium und Aluminium-Zirconium-Mischungen.

2. Mittel gemäß Anspruch 1, **dadurch gekennzeichnet, dass** Methansulfonsäure in einer Gesamtmenge von 0,05 - 5 Gew.-%, bevorzugt 0,1 - 3 Gew.-%, besonders bevorzugt 0,5 - 2,5 Gew.-%, außerordentlich bevorzugt 1 - 2 Gew.-%, enthalten ist, jeweils bezogen auf das Gesamtgewicht des Mittels, wobei die Gesamtmenge an Methansulfonsäure und/oder mindestens einem physiologisch verträglichen Salz der Methansulfonsäure 0,05 - 5 Gew.-%, bevorzugt 0,1 - 3 Gew.-%, besonders bevorzugt 0,5 - 2,5 Gew.-%, außerordentlich bevorzugt 1 - 2 Gew.-%, jeweils bezogen auf das Gesamtgewicht des Mittels, beträgt.

3. Mittel gemäß Anspruch 1 oder 2, **dadurch gekennzeichnet, dass** mindestens ein kosmetisches Öl enthalten ist, das kein Riechstoff und kein ätherisches Öl ist.

4. Mittel gemäß Anspruch 1, 2 oder 3, **dadurch gekennzeichnet, dass** null bis maximal 10 Gew.-% freies Wasser enthalten sind, bezogen auf das Gesamtgewicht des Mittels.

5. Mittel gemäß Anspruch 1, 2 oder 3, **dadurch gekennzeichnet, dass** freies Wasser in einer Gesamtmenge von 15 - 96 Gew.-%, bevorzugt 25 - 80 Gew.-%, besonders bevorzugt 30 - 70 Gew.-%, außerordentlich bevorzugt 40 - 60 Gew.-%, jeweils bezogen auf das Gesamtgewicht des erfindungsgemäßen Mittels, enthalten ist.

6. Mittel nach Anspruch 1, 2, 3, 4 oder 5, **dadurch gekennzeichnet, dass** mindestens ein Emulgator und/oder mindestens ein Tensid enthalten ist.

7. Mittel gemäß Anspruch 1, 2, 3, 5 oder 6, **dadurch gekennzeichnet, dass** es als Öl-in-Wasser-Emulsion vorliegt.

8. Mittel gemäß Anspruch 1, 2, 3, 4, 5 oder 6, **dadurch gekennzeichnet, dass** es als Wasser-in-Öl-Emulsion vorliegt.

9. Produkt gemäß Anspruch 1, 2, 3, 5 oder 6, **dadurch gekennzeichnet, dass** das Mittel als wässrige Lösung vorliegt und bevorzugt weiterhin Ethanol enthält.

10. Produkt gemäß Anspruch 1, 2, 3 oder 4, **dadurch gekennzeichnet, dass** das Mittel als Suspension des ungelösten Antitranspirantwirkstoffs in einem Öl vorliegt.

11. Kosmetisches Mittel nach Anspruch 1, 2, 3, 4, 5, 7, 8, 9 oder 10, **dadurch gekennzeichnet, dass** das Mittel als Stift, Soft Solid, Creme, Roll-on, Dibenzylidenalditol-basiertes Gel, loser Puder oder kompaktierter Puder konfektioniert ist oder auf einem wegwerfbaren Substrat, wie einem Tuch, Pad oder Bausch, aufgetragen vorliegt.

12. Kosmetisches Mittel nach einem der Ansprüche 1 - 11, **dadurch gekennzeichnet, dass** keine Zirconium-haltigen Verbindungen enthalten sind.

13. Verwendung von Methansulfonsäure der Formel (MS-1) 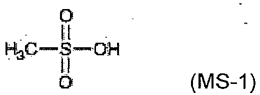 und/oder mindestens einem physiologisch verträglichen Salz der Methansulfonsäure in einem kosmetisch verträglichen Träger, enthaltend
mindestens ein schweißhemmendes Aluminiumsalz, das bevorzugt Zirconium-frei ist, in einer Gesamtmenge von 2 - 40 Gew.-%, bevorzugt 8 - 35 Gew.-%, besonders bevorzugt 10 - 28 Gew.-% und außerordentlich bevorzugt 12 - 20 Gew.-%, wobei sich die Gew.-%-Angaben auf das Gesamtgewicht der kristallwasserfreien und ligandenfreien Aktivsubstanz (USP) in der Zusammensetzung beziehen,
zur Reduzierung oder Verhinderung von durch Antitranspirantien und Antitranspirantwirkstoffe verursachten Textilverfärbungen und/oder Textilflecken, wobei sich die Gew.-%-Angaben jeweils auf das Gesamtgewicht des Mittels beziehen.

14. Verwendung gemäß Anspruch 13, **dadurch gekennzeichnet, dass** das schweißhemmende kosmetische Mittel ein Mittel gemäß einem der Ansprüche 1 - 12 ist.

## Claims

1. A perspiration-inhibiting cosmetic agent for non-aerosol application, containing, in a cosmetically acceptable carrier,
a) at least one perspiration-inhibiting aluminum salt in a total amount of from 2 - 40 wt.%, preferably 8 - 35 wt.%, particularly preferably 10 - 28 wt.% and even more preferably 12 - 20 wt.%, wherein the amounts given in wt.% relate to the total weight of the crystal-water-free and ligand-free active substance (USP) in the agent,
and additionally
b) methanesulfonic acid of the following formula (MS-1) 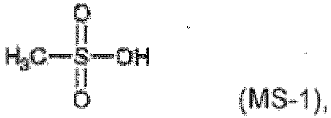
wherein the perspiration-inhibiting aluminum salt is selected from the water-soluble astringent inorganic salts of aluminum and aluminum-zirconium mixtures.

2. The agent according to claim 1, **characterized in that** methanesulfonic acid is contained in a total amount of from 0.05 - 5 wt.%, preferably 0.1 - 3 wt.%, particularly preferably 0.5 - 2.5 wt.%, even more preferably 1 - 2 wt.%, based on the total weight of the agent in each case, the total amount of methanesulfonic acid and/or at least one physiologically acceptable salt of the methanesulfonic acid being 0.05 - 5 wt.%, preferably 0.1 - 3 wt.%, particularly preferably 0.5 - 2.5 wt.%, even more preferably 1 - 2 wt.%, based on the total weight of the agent in each case.

3. The agent according to claim 1 or 2, **characterized in that** at least one cosmetic oil is contained which is not an odorant or an essential oil.

4. The agent according to claim 1, 2 or 3, **characterized in that** zero to a maximum of 10 wt.% free water is contained, based on the total weight of the agent.

5. The agent according to claim 1, 2 or 3, **characterized in that** free water is contained in a total amount of from 15 - 96 wt.%, preferably 25 - 80 wt.%, particularly preferably 30 - 70 wt.%, even more preferably 40 - 60 wt.%, based on the total weight of the agent according to the invention in each case.

6. The agent according to claim 1, 2, 3, 4 or 5, **characterized in that** at least one emulsifier and/or at least one surfactant is contained.

7. The agent according to claim 1, 2, 3, 5 or 6, **characterized in that** it is an oil-in-water emulsion.

8. The agent according to claim 1, 2, 3, 4, 5 or 6, **characterized in that** it is a water-in-oil emulsion.

9. A product according to claim 1, 2, 3, 5 or 6, **characterized in that** the agent is an aqueous solution and also preferably contains ethanol.

10. The product according to claim 1, 2, 3 or 4, **characterized in that** the agent is a suspension of the undissolved antiperspirant active ingredient in an oil.

11. The cosmetic agent according to claim 1, 2, 3, 4, 5, 7, 8, 9 or 10, **characterized in that** the agent is formulated as a stick, soft solid, cream, roll-on, dibenzylideneacetone-based gel, loose powder or compacted powder or is applied to a disposable substrate, such as a cloth, pad or wad.

12. The cosmetic agent according to one of claims 1 to 11, **characterized in that** it does not contain compounds having zirconium.

13. The use of methanesulfonic acid of formula (MS-1) 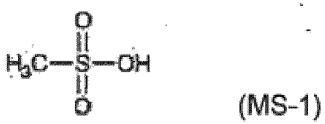 and/or of at least one physiologically acceptable salt of the methanesulfonic acid in a cosmetically acceptable carrier, containing
at least one perspiration-inhibiting aluminum salt, which is preferably free of zirconium, in a total amount of from 2 - 40 wt.%, preferably 8 - 35 wt.%, particularly preferably 10 - 28 wt.% and even more preferably 12 - 20 wt.%, wherein the amounts given in wt.% relate to the total weight of the crystal-water-free and ligand-free active substance (USP) in the composition,
to reduce or prevent textile discolorations and/or textile stains caused by antiperspirants and antiperspirant active ingredients, wherein the amounts given in wt.% relate to the total weight of the agent in each case.

14. The use according claim 13, **characterized in that** the perspiration-inhibiting cosmetic agent is an agent according to one of claims 1 to 12.

## Revendications

1. Agent cosmétique anti-transpirant pour application sans aérosol, contenant, dans un support cosmétiquement acceptable,
a) au moins un sel d'aluminium anti-transpirant en une quantité totale de 2 à 40 % en poids, de préférence de 8 à 35 % en poids, de manière particulièrement préférée de 10 à 28 % en poids et de manière préférée entre toutes de 12 à 20 % en poids, les données exprimées en pourcentage en poids se rapportant au poids total de la substance active anhydre et sans ligand (USP) dans l'agent,
et en plus
b) un acide sulfonique de méthane de formule suivante (MS-1) 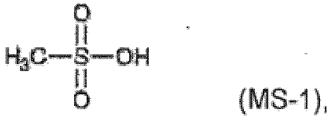
le sel d'aluminium anti-transpirant étant choisi parmi les sels inorganiques astringents hydrosolubles d'aluminium et de mélanges d'aluminium-zirconium.

2. Agent selon la revendication 1, **caractérisé en ce qu'**il contient de l'acide sulfonique de méthane en une quantité totale de 0,05 à 5 % en poids, de préférence de 0,1 à 3 % en poids, de manière particulièrement préférée de 0,5 à 2,5 % en poids, de manière préférée entre toutes de 1 à 2 % en poids, dans chaque cas par rapport au poids total de l'agent, la quantité totale d'acide sulfonique de méthane et/ou d'au moins un sel physiologiquement acceptable de l'acide sulfonique de méthane étant de 0,05 à 5 % en poids, de préférence de 0,1 à 3 % en poids, de manière particulièrement préférée de 0,5 à 2,5 % en poids, de manière préférée entre toutes de 1 à 2 % en poids, dans chaque cas par rapport au poids total de l'agent.

3. Agent selon les revendications 1 ou 2, **caractérisé en ce qu'**il contient au moins une huile cosmétique qui n'est ni une substance aromatique ni une huile essentielle.

4. Agent selon les revendications 1, 2 ou 3, **caractérisé en ce qu'**il contient de zéro à, tout au plus, 10 % en poids d'eau libre par rapport au poids total de l'agent.

5. Agent selon les revendications 1, 2 ou 3, **caractérisé en ce qu'**il contient de l'eau libre en une quantité totale de 15 à 96 % en poids, de préférence de 25 à 80 % en poids, de manière particulièrement préférée de 30 à 70 % en poids, de manière préférée entre toutes de 40 à 60 % en poids, dans chaque cas par rapport au poids total de l'agent selon l'invention.

6. Agent selon les revendications 1, 2, 3, 4 ou 5, **caractérisé en ce qu'**il contient au moins un émulsifiant et/ou au moins un tensioactif.

7. Agent selon les revendications 1, 2, 3, 5 ou 6, **caractérisé en ce qu'**il se présente sous forme d'émulsion d'huile dans l'eau.

8. Agent selon les revendications 1, 2, 3, 4, 5 ou 6, **caractérisé en ce qu'**il se présente sous forme d'émulsion d'eau dans l'huile.

9. Produit selon les revendications 1, 2, 3, 5 ou 6, **caractérisé en ce que** l'agent se présente sous forme de solution aqueuse et contient de préférence en outre de l'éthanol.

10. Produit selon les revendications 1, 2, 3 ou 4, **caractérisé en ce que** l'agent se présente sous forme de suspension de la substance active anti-transpirante non dissoute dans une huile.

11. Agent cosmétique selon les revendications 1, 2, 3, 4, 5, 7, 8, 9 ou 10, **caractérisé en ce que** l'agent est conditionné comme un stick, un solide mou, une crème, une bille, un gel à base de dibenzylidénalditol, une poudre libre ou une poudre compactée, ou se présente de manière appliquée sur un substrat jetable, tel qu'une lingette, un disque ou un tampon.

12. Agent cosmétique selon l'une des revendications 1 à 11, **caractérisé en ce qu'**il ne contient aucun composé contenant du zirconium.

13. Utilisation d'acide sulfonique de méthane de formule (MS-1) 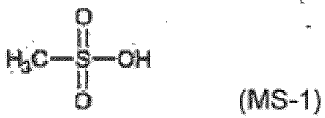 et/ou d'au moins un sel physiologiquement acceptable de l'acide sulfonique de méthane dans un support cosmétiquement acceptable, contenant
au moins un sel d'aluminium anti-transpirant, de préférence exempt de zirconium, en une quantité totale de 2 à 40 % en poids, de préférence de 8 à 35 % en poids, de manière particulièrement préférée de 10 à 28 % en poids et de manière préférée entre toutes de 12 à 20 % en poids, les données exprimées en pourcentage en poids se rapportant au poids total de la substance active anhydre et sans ligand (USP) dans la composition,
pour la réduction ou la prévention des décolorations et/ou des taches sur les textiles causées par des anti-transpirants et des principes actifs anti-transpirants, les données exprimées en pourcentage en poids se rapportant dans chaque cas au poids total de l'agent.

14. Utilisation selon la revendication 13, **caractérisée en ce que** l'agent cosmétique anti-transpirant est un agent selon l'une des revendications 1 à 12.
